(19) Europäisches Patentamt
European Patent Office
Office européen des brevets

(11) EP 1 323 376 A2

(12) EUROPEAN PATENT APPLICATION

(43) Date of publication:
**02.07.2003 Bulletin 2003/27**

(51) Int Cl.[7]: **A61B 5/0452**, G06F 17/00

(21) Application number: **02027517.8**

(22) Date of filing: **06.12.2002**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **27.12.2001 JP 2001397263**
**12.03.2002 JP 2002066782**

(71) Applicant: **Takizawa, Kiyoshi**
**Tokyo102-0084 (JP)**

(72) Inventor: **Takizawa, Kiyoshi**
**Tokyo102-0084 (JP)**

(74) Representative: **Beetz & Partner Patentanwälte**
**Steinsdorfstrasse 10**
**80538 München (DE)**

(54) **Method and apparatus for ECG diagnosis**

(57) The present invention makes a diagnosis of a patient by detecting changes in the state of the patient appearing in an electrocardiogram from a variation in characteristic quantities repeatedly appearing in the electrocardiogram, for example, an R-R interval in an early stage and reliably. The ordinary distribution calculation section 3 receives the electrocardiogram data measured for a period INT1, calculates characteristic quantities repeatedly appearing in the electrocardiogram as time-series data and generates a set C of differences between neighboring elements of the time-series data. The ordinary distribution calculation section 3 further obtains m subsets by repeating m times the processing of randomly collecting n elements from this set C, and calculates an average value a and standard deviation 6 of the set of average values of these m subsets by assuming that the set of average values has normal distribution indicating the ordinary state of the variations in the characteristic quantities. The state detection section 4 calculates a diagnostic set CT from the electrocardiogram data for a period shorter than the period INT1 in the same way as for the above-described set C, and the diagnosis section 5 compares the difference between the average value of this set CT and the average value of the ordinary distribution with the standard deviation 6 and makes a diagnosis.

*FIG.1*

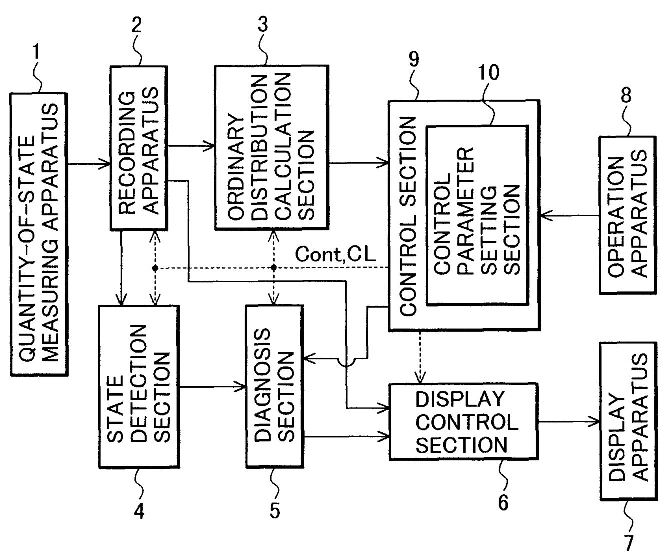


EP 1 323 376 A2

## Description

BACKGROUND OF THE INVENTION

Field of the Invention

The present invention relates to a method and apparatus for diagnosis and a diagnostic program, and more particularly, to a method and apparatus for diagnosis suitable for diagnosing symptoms of patients through electrocardiogram analysis.

Description of the Related Art

Many technologies for diagnosing symptoms of patients by analyzing an electrocardiogram are developed and examples of these technologies include a technology that allows a correct diagnosis by accurately and automatically recognizing characteristic points of individual electrocardiogram waveforms (Japanese Patent Laid-Open No. 8-56914, Japanese Patent Laid-Open No. 9-201344, etc.), a technology that makes a diagnosis by analyzing individual electrocardiogram waveforms (Japanese Patent Laid-Open No. 10-225443, USP5,609,158, USP5,560,368, etc.), a technology that extracts time-series data such as R-R interval from an electrocardiogram waveform and makes a diagnosis by analyzing its time series data (USP5,755,671, Japanese Patent Laid-Open No. 6-54815, etc.), etc. Using these technologies allows more efficient and more speedy diagnosis than diagnosis depending only on a visual check of an electrocardiogram and reduces a possibility of overlooking symptoms.

SUMMARY OF THE INVENTION

The above-described conventional technologies carry out an observation and analysis of an electrocardiogram waveform for a few minutes or over 10 minutes at longest, but it is often the case that data suggesting some abnormality of the heart is not obtained in such a short period. For example, if myocardial infarction occurs, there is a danger of a sudden death in a few hours due to ventricular tachycardia and the probability that arrhythmia will occur in a few days after the occurrence of myocardial infarction reaches 90%. The rate of occurrence of arrhythmia then reduces, but 5 to 10% of patients die within one year. Therefore, it is desirable to acquire and monitor electrocardiogram data for many hours continually for diagnoses of such patients. However, according to the prior arts, a doctor reads the record by the naked eye and makes a diagnosis after the recording is finished, or a doctor extracts short-time data that he/she regards as abnormal from the recorded data and subjects the data to an automatic analysis, etc. For this reason, there is a large time delay after measurement until the diagnosis result is obtained, and the prior arts are insufficient in prognosticating changes in a symptom or imminent danger. Furthermore, since the prior arts involve judgments by the naked eye of the doctor, there is a problem that oversight is likely to occur.

More generally, in the case that repetitive vibration accompanied by complicated swinging in a physical quantity of an object occurs, it is required that reliable and speedy diagnosis for soundness of such object should be carried out through continuous acquisition an automatic analysis of the physical quantity.

It is an object of the present invention to provide a diagnostic method capable of monitoring respective variations in the physical quantity of an object all the time and auto-detecting characteristic changes of the object speedily and reliably, and in particular, to provide a method and apparatus for diagnosis and a diagnostic program, which observes and analyzes continually an electrocardiogram of a patient for a long period of time and is capable of detecting speedily and automatically any abnormality that occurs.

In order to attain the above-described object, the present invention provides a diagnostic method for performing diagnosis and a diagnostic apparatus to realize the diagnostic method, including the steps of incorporating electrocardiogram data measured by an electrocardiograph apparatus for a given first period as distribution calculation data, detecting characteristic quantities repeatedly appearing in the electrocardiogram data from the distribution calculation data to generate time-series data, generating a difference set whose elements consist of absolute values of differences between neighboring data pieces of this time-series data, generating m subsets each of which consists of randomly collected n elements from this difference set (n, m: positive integers), calculating each average value of the elements of the m each subset to generate an average value set as a set having the ordinary distribution of the difference set, and calculating average value and standard deviation of the average value set by assuming that the average value set has normal distribution, and

incorporating the electrocardiogram data measured by the electrocardiograph apparatus for a second period which is shorter than the first period as diagnostic data, detecting characteristic quantities repeatedly appearing in the electrocardiogram data from the diagnostic data to generate time-series data, generating a difference set whose elements consist of absolute values of differences between neighboring data pieces of this time-series data, and comparing

the absolute value of the difference between the average value of the elements of this difference set and the average value of the ordinary distribution with a diagnostic level obtained by multiplying a given diagnostic coefficient by the standard deviation.

The present invention also provides a diagnostic method for performing diagnosis and a diagnostic apparatus to realize the diagnostic method, including the steps of incorporating electrocardiogram data measured by an electrocardiograph apparatus for a given first period as distribution calculation data, detecting characteristic quantities repeatedly appearing in the electrocardiogram data from the distribution calculation data to generate time-series data, generating a difference set whose elements consist of absolute values of differences between neighboring data pieces of this time-series data, generating m subsets each of which consists of randomly collected n elements from this difference set (n, m: positive integers), calculating each average value of the elements of the m each subset to generate an average value set as a set having the ordinary distribution of the difference set, and calculating average value and standard deviation of the average value set by assuming that the average value set has normal distribution,

repeatedly setting a second period which is shorter than the first period with the passage of time, incorporating the electrocardiogram data measured by the electrocardiograph apparatus as diagnostic data for every second period, detecting characteristic quantities repeatedly appearing in the electrocardiogram data from each diagnostic data to generate time-series data respectively, and generating difference sets each of which elements consist of absolute values of differences between neighboring data pieces of corresponding time-series data, and

displaying an average value variation graphic that plots average values of the respective difference sets in association with times representing the second period corresponding to the difference sets together with a diagnostic level obtained by multiplying a given diagnostic coefficient by the standard deviation on a displaying means, displaying, when an arbitrary time on this average value variation graphic is specified through an operating means, an electrocardiogram waveform indicating a time variation of the electrocardiogram data in a time zone including the specified time on the displaying means so that the average value variation graphic or the electrocardiogram waveform is able to be observed for diagnosis.

The present invention also provides a diagnostic method for performing diagnosis and a diagnostic apparatus to realize the diagnostic method, including the steps of incorporating electrocardiogram data measured by an electrocardiograph apparatus for a given first period as distribution calculation data, detecting characteristic quantities repeatedly appearing in the electrocardiogram data from the distribution calculation data to generate time-series data, generating a difference set whose elements consist of absolute values of differences between neighboring data pieces of this time-series data, generating m subsets each of which consists of randomly collected n elements from this difference set (n, m: positive integers), calculating each average value of the elements of the m each subset to generate an average value set as a set having the ordinary distribution of the difference set, and calculating average value and standard deviation of the average value set by assuming that the average value set has normal distribution,

repeatedly setting a second period which is shorter than the first period with the passage of time, incorporating the electrocardiogram data measured by the electrocardiograph apparatus as diagnostic data for every second period, detecting characteristic quantities repeatedly appearing in the electrocardiogram data from each diagnostic data to generate time-series data respectively, and generating difference sets each of which elements consist of absolute values of differences between neighboring data pieces of corresponding time-series data, and

counting during a given third period the number of times this average value of each difference set exceeds a diagnostic level obtained by multiplying a diagnostic coefficient by the standard deviation, and comparing the number of times with a given number of times for diagnosis.

The present invention also provides the above-described diagnostic method and diagnostic apparatus characterized in that calculations of an average value and standard deviation of the ordinary distribution from the distribution calculation data are repeated by periodically changing the period of incorporating the distribution calculation data and the average value and the standard deviation of the ordinally distribution are periodically updated.

The present invention also provides the above-described diagnostic method and diagnostic apparatus characterized in that electrocardiogram data for ordinally distribution calculation and diagnosis is incorporated via a network.

The present invention also provides a diagnostic apparatus equipped with a center apparatus and one or a plurality of measuring terminal apparatuses connected to this center apparatus via a network,

each of the measuring terminal apparatuses including

an electrocardiograph apparatus,

parameter setting means for setting at least a first period or a second period which is shorter than this first period a positive integer parameter n, a positive integer parameter m and a diagnostic coefficient,

ordinally distribution calculating means for incorporating electrocardiogram data measured by the electrocardiograph apparatus as distribution calculation data for the first period set in the parameter setting means, detecting characteristic quantities repeatedly appearing in the electrocardiogram from the distribution calculation data to generate time-series data, generating a difference set whose elements consist of absolute values of differences between neighboring data pieces of this time-series data, generating as many subsets as parameter m set in the parameter setting

means each of which consists of n elements randomly collected from the difference set where n is the parameter set in the parameter setting means, calculating each average value of the elements of the each subset to generate an average value set as a set having the ordinary distribution of the difference set, and calculating average value and standard deviation of the average value set by assuming that the average value set has normal distribution,

state detecting means for repeatedly setting the second period set in the parameter setting means with the passage of time, incorporating the electrocardiogram data measured by the electrocardiograph apparatus as diagnostic data for every second period, detecting characteristic quantities repeatedly appearing in the electrocardiogram from each diagnostic data to generate time-series data respectively, generating difference sets each of which elements consist of absolute values of differences between neighboring data pieces of corresponding time-series data, and calculating an average value of each difference set,

diagnostic means for performing diagnosis by comparing the absolute value of the difference between the average value calculated by the state detecting means and the average value of the ordinary distribution with a diagnostic level obtained by multiplying the diagnostic coefficient set in the parameter setting means by the standard deviation,

a communication interface,

controlling means for controlling, during ordinary operation mode, so that the diagnostic result of the diagnostic means is sent to the center apparatus via the communication interface and network by operating the ordinary distribution calculating means, the state detecting means and the diagnostic means according to the parameters set in the control parameter setting means, and controlling, when a command is sent from the center apparatus, so that one or both of the electrocardiogram data from the electrocardiograph apparatus and the average value calculated by the state detecting means for every second period are sent to the center apparatus via the communication interface and network according to the content of the command, and

that the center apparatus includes

control parameter setting means for setting control parameters used by each measuring terminal apparatus,

display controlling means for controlling the displaying means,

a communication interface,

inputting means, and

controlling means for sending parameters which are set in the control parameter setting means through said inputting means to the corresponding measuring terminal apparatus via the communication interface and network so that the parameters are set in the control parameter setting means of the measuring terminal apparatus, and sending, when a command is input from the inputting means to one of the measuring terminal apparatuses, the command to controlling means of the measuring terminal apparatus via the communication interface and network, controlling the display controlling means during ordinary operation mode so as to display the diagnostic result sent from each measuring terminal apparatus, and controlling the display controlling means, when one or both of the electrocardiogram data and time-series data of the average values are sent according to the command, so as to display the data.

The present invention also provides a diagnostic program for allowing a computer to execute:

a first step for of incorporating electrocardiogram data measured by an electrocardiograph apparatus a given first period as distribution calculation data, detecting characteristic quantities repeatedly appearing in the electrocardiogram data from the distribution calculation data to generate time-series data, generating a difference set whose elements consist of absolute values of differences between neighboring data pieces of this time-series data, generating m subsets each of which consists of randomly collected n elements from this difference set (n, m: positive integers), calculating each average value of the elements of each subset to generate an average value set as a set having the ordinary distribution of the difference set, and calculating average value and standard deviation of the average value set by assuming that the average value set has normal distribution, and

a second step of incorporating the electrocardiogram data measured by the electrocardiograph apparatus for a second period which is shorter than the first period as diagnostic data, detecting characteristic quantities repeatedly appearing in the electrocardiogram data from the diagnostic data to generate time-series data, generating a difference set whose elements consist of absolute values of differences between neighboring data pieces of this time-series data, comparing the absolute value of the difference between the average value of this difference set and the average value of the ordinary normal distribution with a diagnostic level obtained by multiplying a given diagnostic coefficient by the standard deviation, and displaying the comparison result on a displaying means.

The present invention also provides a diagnostic program for allowing a computer to execute:

a first step of incorporating electrocardiogram data measured by an electrocardiograph apparatus for a given first period as distribution calculation data, detecting characteristic quantities repeatedly appearing in the electrocardiogram data from the distribution calculation data to generate time-series data, generating a differential set whose elements consist of absolute values of differences between neighboring data pieces of this time-series data, generating m subsets each of which consists of randomly collected n elements from this difference set (n, m: positive integers), calculating each average value of the elements of each subsets to generate an average value set as a set having the ordinary distribution of the difference set, and calculating average value and standard deviation of the average value set by assuming that the average value set has normal distribution,
a second step of repeatedly setting a second period which is shorter than the first period with the passage of time, incorporating the electrocardiogram data measured by the electrocardiograph apparatus as diagnostic data for every second period, detecting characteristic quantities repeatedly appearing in the electrocardiogram data from each diagnostic data to generate time-series data respectively, and generating a difference sets each of which consists of absolute values of differences between neighboring data pieces of corresponding time-series data, and
a third step of displaying an average value variation graphic that plots average values of the respective difference sets in association with times representing the second period corresponding to the difference sets together with a diagnostic level obtained by multiplying a given diagnostic coefficient by the standard deviation on a displaying means, and displaying, when an arbitrary time on this average value variation graphic is specified through an operating means, an electrocardiogram data waveform indicating a time variation of the electrocardiogram in a time zone including the specified time on the displaying means.

The present invention also provides a diagnostic program for allowing a computer to execute:

a first step of incorporating electrocardiogram data measured by an electrocardiograph apparatus for a given first period as distribution calculation data, detecting characteristic quantities repeatedly appearing in the electrocardiogram data from the distribution calculation data to generate time-series data, generating a difference set whose elements consist of absolute values of differences between neighboring data pieces of this time-series data, generating m subsets each of which consists of randomly collected n elements from this difference set (n, m: positive integers), calculating each average value of the elements of each subset to generate an average value set as a set having the ordinary distribution of the difference set, and calculating an average value and standard deviation of the average value set by assuming that the average value set has a normal distribution,
a second step of repeatedly setting a second period which is shorter than the first period with the passage of time, incorporating the electrocardiogram data measured by the electrocardiograph apparatus as diagnostic data for every second period, detecting characteristic quantities repeatedly appearing in the electrocardiogram data from each diagnostic data to generate time-series data respectively, and generating a difference sets each of which consists of absolute values of differences between neighboring data pieces of corresponding time-series data, and
a third step of counting during a given third period the number of times this average value of each difference set exceeds a diagnostic level obtained by multiplying a diagnostic coefficient by the standard deviation, and displaying the result of comparing this count with a given number of times for diagnosis on a displaying means.

The present invention provides the above-described diagnostic program characterized in that the first step includes the steps of repeating calculations of an average value and standard deviation of the ordinary distribution using the distribution calculation data by periodically changing the period of incorporating the distribution calculation data, and periodically updating the average value and the standard deviation of the ordinary distribution.
The present invention also provides a diagnostic method for performing diagnosis of an object including the steps of:

incorporating measured quantity of state of the object for a first given period as distribution calculation data, detecting characteristic quantities repeatedly appearing in the quantity of state from the distribution calculation data to generate time-series data, generating a difference set whose elements consist of absolute values of differences between neighboring data pieces of this time-series data, generating m subsets each of which consists of randomly collected n elements from this difference set (n, m: positive integers), calculating each average value of the elements of each subset to generate an average value set as a set having the ordinary distribution of the difference set, and calculating average value and standard deviation of the average value set by assuming that the average value set has a normal distribution,
incorporating measured quantity of state of the object for a second period which is shorter than the first period as diagnostic data, detecting characteristic quantities repeatedly appearing in the quantity of state from the diagnostic data to generate time-series data, generating a difference set whose elements consist of absolute values of differences between neighboring data pieces of this time-series data, and comparing the absolute value of the difference between the average value of the elements of the difference set and the average value of the ordinary distribution with a diagnostic level obtained by multiplying a given diagnostic coefficient by the standard deviation.

BRIEF DESCRIPTION OF THE DRAWINGS

- FIG. 1 is a block diagram showing a configuration example of a diagnostic apparatus according to the present invention;
- FIG. 2 is a flow chart showing processing of an ordinary distribution calculation apparatus;
- FIG. 3 is a flow chart showing processing of a state detection apparatus;
- FIG. 4 is another flow chart showing processing of the state detection apparatus;
- FIG. 5 illustrates a diagnostic data acquisition timing;
- FIG. 6 is a flow chart showing processing of a display control apparatus;
- FIG. 7 is a block diagram showing another configuration example of the diagnostic apparatus according to the present invention;
- FIG. 8 is a block diagram showing another configuration example of the diagnostic apparatus according to the present invention;
- FIG. 9 is a schematic view of a basic electrocardiogram waveform; and
- FIG. 10 illustrates the S wave.

DETAILED DESCRIPTION OF THE PREFERRED EMBODIMENTS

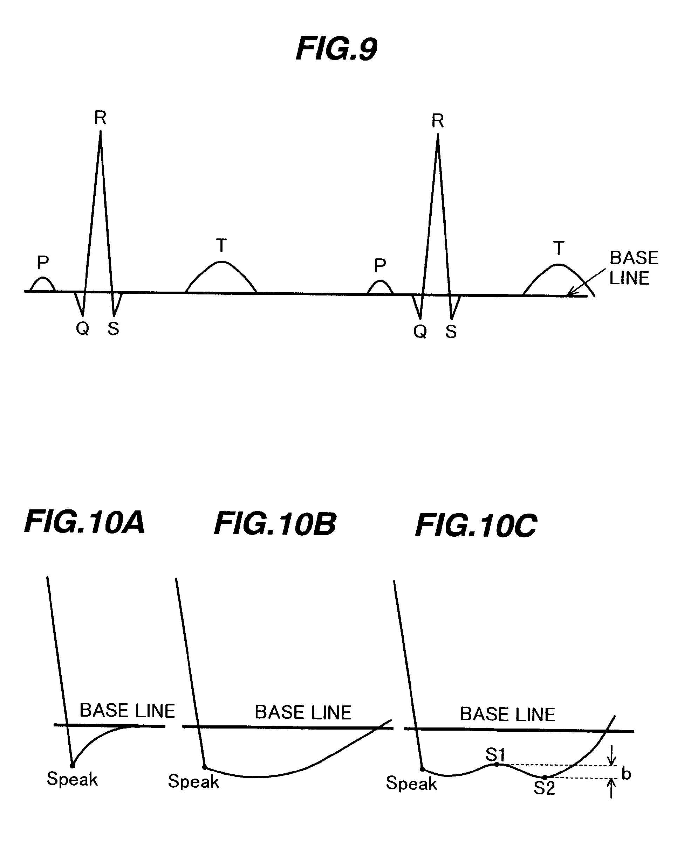

Embodiments of the present invention will be explained in detail below using a diagnosis by an electrocardiogram analysis as an example. FIG. 9 is a schematic view of a basic electrocardiogram waveform in which P, Q, R, S and T waves are observed as waves expressing states of various apparatuses of a heart. Various characteristic quantities of such waveforms are often used for an electrocardiogram analysis. Examples of these characteristic quantities include an R-R interval indicating a distance between peaks of neighboring R waves (heart beat interval), an ST falling apparatus area indicating an area of the S wave, an evaluation value W indicating a characteristic of the waveform of this ST falling apparatus, or a QS interval indicating the width from the start of the Q wave to the end of the S wave, etc.

Here, the ST falling apparatus (S wave) will be explained. When there is no abnormality, the waveform after point "$S_{peak}$" of the S wave is upward convex and the waveform approaches the base line relatively early as shown in FIG. 10A. However, with the presence of an ischemic disorder, a downward convex curve appears and at the same time, the waveform approaches the base line late and the area of the S wave also increases as shown in FIG. 10B and FIG. 10C. Especially in the case of FIG. 10C, there are two extreme values S1 and S2 between point $S_{peak}$ and the base line, which indicates more serious state than the case in FIG. 10B. Suppose the above-described evaluation value W is given by the following formula using a difference between these extreme values b = |S1-S2| (mV millivolt).

[Formula 1]

$$W = \begin{cases} 0, \text{ when the S wave is upward convex as shown in FIG. 10A} \\ 1, \text{ when the S wave is downward convex, without two extreme values as shown in FIG. 10B} \\ b \langle mV \rangle \times 10, \text{ when the S wave is downward convex, with two extreme values as shown in FIG. 10C} \end{cases}$$

Furthermore, when the evaluation value of the ST falling area is expressed with "U", U is given by the following formula in the sense that the cases in FIG. 10B and 10C are emphasized.

[Formula 2]

$$U = \text{ST falling area} < mV \cdot sec > X \begin{cases} 0, & \text{in the case of FIG. 10A} \\ 1, & \text{in the case of FIG. 10B} \\ 4, & \text{in the case of FIG. 10C} \end{cases}$$

Each of the characteristic quantities of the electrocardiogram waveform illustrated above construct time-series data that takes one value per one heart beat. This time-series data itself generally has different values, which differs, from one patient to another. For example, when the R-R interval of a plurality of healthy people who stay equally tranquil is measured, the average R-R interval may vary from one person to another; for example, it may be 1 sec (heart rate per minute of 60) for one person, while it may be 6/7 sec (heart rate per minute of 70) for another person. On top of it, the R-R interval per beat is not completely constant but varying minutely. Furthermore, after taking exercise or when a symptom changes, the average value of R-R interval as well as its minute variation may change. The present invention is intended to detect changes in a symptom by detecting changes in such characteristic quantities.

An analysis method used in the present invention to detect such changes on the characteristic quantities will be explained taking electrocardiogram data as an example. First, the present invention collects digitalized electrocardiogram data of a target patient for a long period of time, for example, for several days. This collection period is denoted by INT1, and the collected electrocardiogram data between the period INT1 is expressed as a set A. Suppose now, for example, a signal from an electrocardiogram monitor is sampled every 1 ms and each sampled value is digitalized into a code of 12 bits. Then the number of data Na (number of samples) per 1 sec is 1000, and the amount of information in bytes (B) Ma is 12 kbits = 1.5 kB. This means the number of data Na is 86.4 millions and the amount of information Ma is approximately 130 MB per one day. Therefore, the amount of information Ma of the set A is about several hundreds of MB when it is data for several days.

Characteristic quantities (e.g., R-R interval) calculated from a electrocardiogram data set A collected as shown above form a time-series data, which is expressed as a set $B\{x_j\}$, j=0, 1, where each element Xj is a characteristic quantity ordered in time. Furthermore, from this set B, a set of absolute values $y_j$ of a difference between neighboring characteristic quantities is calculated according to:

[Formula 3]

$$y_j = |x_j - x_{j-1}|$$

and this difference set is expressed as $C\{y_j\}$, j=1, 2, ···. The number of data Nc of this set is 100,800 per day when the heart rate is 70/min and the amount of information Mc is approximately 1.5 MB per day assuming that a difference data $y_j$ has 12 bits.

Then, the distribution of the set C(d.h. distribution of the elements of the set C) is calculated, assuming this is the ordinary distribution representing ordinary state of a patient. For this purpose, an operation of generating a subset composed of n pieces (e.g., 1000) of data randomly extracted from the difference set $C\{y_j\}$ is repeated m times and suppose those subsets are $C_\alpha = \{y_{\alpha 1}, y_{\alpha 2}, \cdots y_{\alpha n}\}$, $\alpha$ = 1 to m (e.g., m = 3000). However, random extractions of subsets $C_\alpha$, $\alpha$ = 1 to m are performed in such a way that there is no correlation with one another. Then, when an average value $e_\alpha$ of elements of subset $C_\alpha$ is calculated as:

[Formula 4]

$$e_\alpha = (y_{\alpha 1} + y_{\alpha 2} + \cdots + y_{\alpha n})/n, \ \alpha = 1 \text{ to } m$$

it is well known that the distribution of the set of average value $e_\alpha$ can be regarded as a normal distribution if the above-described numbers of samples n and m are sufficiently large no matter what distribution of the original set A may be. Here, assuming that the set ND of the average value $e_\alpha$ calculated by (Formula 4) follows a normal distribution, its average value $\mu$ and standard deviation $\sigma$ are calculated as follows:

[Formula 5]

$$\mu = (1 / m) \sum_{\alpha=1}^{m} e_{\alpha}$$

$$\sigma = \left\{(1 / m) \sum_{\alpha=1}^{m} (e_{\alpha} - \mu)^{2}\right\}^{1/2}$$

Even if any temporary variations in characteristic quantities such as a heart rate and R-R interval occur because of the physical movement or symptom of the patient, the distribution ND ($\mu$, $\sigma$) of the average value $e_{\alpha}$ calculated as shown above can be considered to express an average state of the cardiac activity of the patient if the electrocardiogram data set A is large enough and the data size n of subset $C_{\alpha}$ and the number of subsets m are sufficiently large. Therefore the distribution ND is regarded as the ordinary distribution mentioned above, an can be used as the reference for detecting variations of the cardiac activity. By the way, it is desirable to calculate and update this ordinary distribution for the period INT1 until that time point, such as once every few days or once a week and the length of the period INT1 at every update time point can be changed.

Then, the diagnostic method using the ordinary distribution calculated as shown above will be explained. First, suppose electrocardiogram data set AT for a period INT2 which is sufficiently shorter than the set A data collection period INT1 is incorporated for a diagnosis and a set of (p+1) characteristic quantities $BT_k = \{x_k, x_{k+1}, \cdots x_{k+p}\}$ is calculated from this data set. Then, from this set $BT_k$, a difference set $CT_k = \{y_{k+1}, y_{k+2} \cdots y_{k+p}\}$ composed of p data pieces is obtained according to (Formula 3) and its average value $\mu_k$ is calculated as follows:

[Formula 6]

$$\mu_k = (1 / p) \sum_{j=1}^{p} y_{k+j}$$

Then, the average value deviation Zk, which is the distance between the average value $\mu_k$ calculated from diagnosis data set AT and the pre-calculated average value $\mu$ of the ordinary distribution, is calculated as follows;

[Formula 7]

$$Z_k = |\mu_k - \mu|$$

Then this value is compared with the standard deviation $\sigma$ of the ordinary distribution ND. For example, if $|Z_k| > 3\sigma$, it is decided that some abnormal symptom change has occurred. Such a change can be detected by calculating the set $BT_k$ of characteristic quantities from the electrocardiogram data AT of the period INT2, calculating the difference set $CT_k$ thereof, and then calculating average value deviation $Z_k$ through calculations of (Formula 6) and (Formula 7) and comparing this with the standard deviation $\sigma$ of the ordinary distribution, and this series of calculations can be executed in an extremely short time. Therefore, if the above detection is carried out, for example, every one minute, it is possible to quickly detect a change of symptom of a patient every one minute.

When the number of data p of the difference set $CT_k$ is small, it is sometimes difficult to make a precise diagnosis using only one $\mu_k$ value. In such a case, it is possible to calculate r difference sets $CT_{k1}$, $CT_{k2} \cdots CT_{kr}$, for example, every one minute, and given the alarm when the number of times a difference between their average values $\mu_{k1}$, $\mu_{k2}$, $\cdots\mu_{kr}$ and the average value $\mu$ of the ordinary distribution becomes $3\sigma$ or greater is a pre-defined value r0 or greater.

The above-described diagnostic method using electrocardiogram data is intended to make a diagnosis by expressing a ordinary state of the patient with a ordinary (normal) distribution calculated from electrocardiogram data acquired over a long period INT1 and evaluating at every inspection time point the difference between the average

value $\mu_k$ of the difference set $CT_k$ calculated from the electrocardiogram data of the relatively shorter period INT2 or average values $\mu_{k1}$, $\mu_{k2}$ ··· of a plurality of difference sets $CT_{k1}$, $CT_{k2}$, ··· and the average value μ of the ordinary distribution. Furthermore, since calculation processing at every inspection time point can be performed virtually in real time, it is possible to monitor the heart condition of the patient all the time and automatically detect any abnormality at an early stage. Thus, this diagnostic result allows the doctor, etc. to clearly comprehend the change in the heart condition even if the change is transient and carry out appropriate examinations and cure in consideration of the heart condition of the relevant patient. This is useful to make an early diagnosis, give advice to the patient or recommend preventive actions based on the diagnostic results for not only patients who are considered to already have cardiac disease but also people who are considered healthy.

If the inspection data acquisition period INT2 is a period of time during which a characteristic quantity of, for example, 1000 can be obtained (when the heart rate is 60, INT2 is approximately 17 minutes), even if there is some error in the detection of the characteristic quantity, the influence of the error on the average value $\mu_k$ calculated is extremely small and the influence on the diagnostic result is likewise small unless the probability of the error detection is significantly high. The same applies to the ordinary distribution calculated by statistical handling of a larger set. Thus, compared to the conventional diagnostic method using short-time electrocardiogram data, the probability that noise or treatment errors in the process of acquisition of electrocardiogram data and detection of characteristic quantities extracted will affect the diagnostic results is extremely low, and the present invention can provide a diagnostic method applicable to an environment in which there is a certain degree of noise.

The above-described analysis method is likewise applicable to a diagnosis of structures, etc. that produce vibration accompanied by complicated swinging. In such a case, it is only necessary to incorporate a quantity of state of the object instead of electrocardiogram data and carry out a similar analysis using the characteristic quantities related to expected changes of state and it is possible to obtain effects similar to those of the electrocardiogram data.

A configuration of a diagnostic apparatus using the above-described analysis method will be explained below. FIG. 1 is a block diagram showing a configuration example of a diagnostic apparatus according to the present invention, which is constructed of a quantity of state measuring apparatus 1 that measures quantities of state of an object consecutively, digitalizes and outputs the quantities of state, a recording apparatus 2 capable of recording the quantities of state from the apparatus 1 for a period of at least INT1, an ordinary distribution calculation apparatus 3 that calculates an average value μ and standard deviation σ of the aforementioned ordinary distribution ND from the quantities of state stored in the recording apparatus 2, a state detection apparatus 4 that calculates a differential set $CT_k$ and average value $\mu_k$ of the characteristic quantities, a diagnosis apparatus 5 that makes a diagnosis of the state at that time using the average value $\mu_k$ calculated by the detection apparatus 4 and parameters μ and σ of the ordinary distribution ND, a display control apparatus 6 that controls displays of the diagnostic result of the diagnosis apparatus 5 and the quantities of state data on the recording apparatus 2, etc., a display apparatus 7, an operation apparatus 8 and a control apparatus 9 that controls operations of various apparatuses according to instructions from the operation apparatus 8.

In this configuration, when a quantity of state is electrocardiogram data, the quantity-of-state measuring apparatus 1 can be an electrocardiograph apparatus which can provide with A/D-converted digital quantities of analog electrocardiogram data measured by the apparatus consecutively for a long period of time. On the other hand, when a quantity of state is a displacement at a measuring point of a mechanical structure, the quantity-of-state measuring apparatus 1 is a vibration gauge which measures the displacement and outputs it as digital data. In any case where a quantity of state needs to be digitalized, suppose the measuring apparatus 1 incorporates a clock generator supplying clock signals for data sampling and coding. All of the recording apparatus 2, normal distribution calculation apparatus 3, state detection apparatus 4, display control apparatus 6 and control apparatus 9 are devices that carry out digital processing and it is possible to construct each of these devices with a DSP, etc. or with a general-purpose processing apparatus such as a personal computer executing a suitable program.

Control apparatus 9 is provided with a control parameter setting apparatus 10 to control operations of various apparatuses. This control parameter setting apparatus 10 allows the operation apparatus 8 to set an ordinary distribution ND calculation cycle T1, data acquisition period INT1 used for one calculation of the ordinary distribution, number m of subsets $C_\alpha$ extracted from characteristic quantity difference set $C\{y_j\}$, size n of each subset, electrocardiogram data acquisition cycle T2 and acquisition period INT2 by the state detection apparatus 4, diagnostic coefficient for determining one or a plurality of diagnostic levels as references to display alarms, etc. by a comparison at the diagnosis apparatus 5, number r of subsets and number of times r0 as references to display alarms when a diagnosis is made using an average value of a plurality of subsets $CT_k$, number of ordinary distribution calculation times in the above-described cycle T1 or calculation stopping time Q1, number of times a diagnosis is automatically made in the above-described cycle T2 or diagnosis stopping time Q2, etc. The control apparatus 9 has a function of generating and supplying a clock CL necessary to operate various apparatuses. The control apparatus 9 not only gives parameters set in the control parameter setting apparatus 10 to various apparatuses but also controls operations of various apparatuses according to a start instruction from the operation apparatus 8 based on a control signal "cont" and the clock CL.

By the way, the recording apparatus 2 needs to be controlled so that a data write from the quantity of state

measuring apparatus 1 does not collide with a data read from the ordinary distribution calculation section 3, quantity of state detection section 4 and display control section 6. Also the sampling clock of the quantity of state measuring apparatus 1 and the clock CL from the control section 9 are usually independent of each other and may have quite different clock frequencies. However, if the recording apparatus 2 is constructed of a hard disk apparatus and its control circuit, the amount of information to be read or written is sufficiently small considering the read/write speed of the recording apparatus, and therefore it is easy to avoid the above-described collision by providing a buffer for the control circuit incorporated in the recording apparatus 2 to control the read/write.

The operation of apparatus shown in FIG. 1 will be explained below, assuming that the quantity of state measuring apparatus 1 is an electrocardiograph apparatus. FIG. 2 is a flow chart showing the processing in the ordinary distribution calculating section 3. In Fig. 2, after a control variable $\beta$ is set to 1 (step 200), it is decided whether it is time to calculate a ordinary distribution or not (step 201). This calculation time corresponds to the time at which a first start instruction is given from the operation apparatus 8 or when the calculation cycle T1 set in the control parameter setting section 10 has passed from the previous calculation time. When it is time to calculate a ordinary distribution (when the decision result in step 201 is YES), the electrocardiogram data (set A) for the period INT1 set in the control parameter setting section 10 before that time point is incorporated from the recording apparatus 2 (step 202), and the time-series data $B\{x_j\}$ of the characteristic quantities as analysis target is calculated from this set A (step 203). When the characteristic quantity is R-R interval, for example, this calculation processing consists of detecting a peak points of R waves and calculating their intervals. Such calculation for any characteristic quantity of electrocardiogram is performed easily by using a known method. When an ST wave is used, (Formula 1) and (Formula 2), etc. can also be used, but details thereof will be omitted here.

When all the time-series data $B\{x_j\}$ of the characteristic quantities during the period INT1 is obtained, then the set $C\{y_j\}$ of the absolute values of differences of the neighboring elements of the time-series data $B\{x_j\}$ is calculated according to (Formula 3) (step 204) and a control variable $\alpha$ is set to 1 (step 205). Uniting steps 203 and 204 by calculating a difference $y_j=|x_j-x_{j-1}|$ immediately after the characteristic quantity $x_j$ is obtained would improve the processing efficiency, but these steps are separated here to make the relationship with the aforementioned diagnostic method easier to understand. Then, the number of parameters m set in the control parameter setting section 10 is compared with the above-described control variable $\alpha$ and if $\alpha \leq m$ (when the comparison result in step 206 is YES), n elements are randomly extracted from the set $C\{y_i\}$ to make a subset $C_\alpha(y_{\alpha 1}, y_{\alpha 2}, \cdots y_{\alpha n})$ (step 207) and an average value $e_\alpha$ of the n elements is calculated according to (Formula 4) (step 208). Then, the control variable $\alpha$ is incremented by +1 (step 209) and the process moves back to step 206. The processing in these steps 206 to 209 is repeated m times, m subsets $C_\alpha$, $\alpha$ = 1 to m are extracted from the set $C\{y_i\}$ and their average values $e_\alpha$, $\alpha$= 1 to m are calculated. When, $\alpha > m$ is satisfied (when the comparison result in step 206 is NO), an average value $\mu$ and a standard deviation $\sigma$ of the m average values $e_\alpha$, $\alpha$ =1 to m, that is, a ordinary distribution ND ($\mu$, $\sigma$) are calculated by (Formula 5) (step 210).

Then, when the control variable $\beta$ is smaller than the parameter Q1 set in the control parameter setting section 10 (when the comparison result in step 211 is Yes), $\beta$ is incremented by +1 (step 212) and the process moves back to step 201, but if $\beta \geq Q1$ (when the comparison result in step 211 is NO), the process ends here. In the above-described processing, if the control variable Q1 is set to 1, this means that only one time calculation of the ordinary distribution is specified from the operation apparatus 8, and this is effective for a system check or preliminary comprehension of the condition of the patient by changing parameters m, n, INT1, etc. Furthermore, when calculations are continued until the operation apparatus 8 sends a stop instruction without particularly setting the number of times the ordinary distribution is calculated or the end time of calculations, it is possible to delete steps 200 and 212 in FIG. 2 and decide whether there is a stop instruction or not in step 211 and then return to step 201 or end the procedure. Or it is also possible to set the parameter Q1 to a sufficiently large value without changing the procedure in FIG. 2.

As the method for randomly extracting n elements in step 207, the function strand (·) provided in the C programming language or the logic equivalent thereto for example, is available. If each pseudo-random number series between 1 to NC (number of data pieces of differential set $C\{y_j\}$) is generated by:

[Formula 8]

strand (time (null))

where the CPU clock that time is used as an argument time (null), and if the n elements $y_j$s having suffix j which belongs to the pseudo-random number series are extracted from the set $C\{y_j\}$, there is almost no correlation in the way of selection of elements between any two subsets $C_\alpha$ and $C_\gamma$ ($\alpha \neq \gamma$) generated at different CPU time.

FIG. 3 is a flow chart showing the processing in the state detection section 4 and diagnosis section 5. After a control variable $\beta$ is set to 1 (step 300), it is decided whether it is time to inspect to detect changes in the characteristic quantities or not (step 301). Like the ordinary distribution calculation time, this time also corresponds to the time at

which the first start instruction is given from the operation apparatus 8 or the time at which the calculation cycle T2 set in the control parameter setting section 10 has passed after the previous detection. When it is time to inspect, the electrocardiogram data AT for the period INT2 set in the control parameter setting section 10 before that time point is incorporated from the recording apparatus 2 (step 302), and the time-series data $BT_k\{x_j\}$, j=k to k+p of the characteristic quantities to be analyzed is calculated from the electrocardiogram data (step 303). When the time-series data $BT_k\{x_j\}$ of the characteristic quantities is obtained, a difference set $CT_k\{y_j\}$, j=k+1 to k+p is obtained as the inspection data by substituting the time-series data elements $x_j$, j=k, k+1, ... into (Formula 3) (step 304),and an average value $\mu_k$ of this differential set is calculated from (Formula 6) (step 305). By the way, uniting the processing in steps 303 and 304 will improve the processing efficiency as in the case of the calculation of the ordinary distribution. Then, an average value deviation $Z_k = |\mu_k-\mu|$ ($\mu$: average value of ordinary distribution) is calculated by (Formula 7), and this $Z_k$ is compared with the standard deviation $\sigma$ of the ordinary distribution and the comparison result is sent to the display control section 6 (step 306). For example, if two diagnostic coefficients 3 and 5 is set in the parameter setting section 10 and L1=3$\sigma$, L2=5$\sigma$ are used as the diagnostic levels, a signal indicating "Attention" is sent to the display control section 6 when $Z_k$>L1, and a signal indicating "Danger" is sent to the display control section 6 when $Z_k$>L2. Then, when the control variable $\beta$ is smaller than the parameter Q2 (when comparison result in step 307 is Yes), the process returns to step 301 and when $\beta \geq$Q2, the process ends.

In the above-described processing in FIG. 3, a diagnosis is made for every one difference set $CT_k$, but when a diagnosis is made by calculating an average value for a plurality of difference sets and detecting the number of those average values that exceed a predetermined number of times, the processing in the flow chart shown in FIG. 4 is carried out. In this case, three control variables $\beta$, $\gamma$, $\beta1$ and $\beta0$ counter variables $\Gamma_1$ to $\Gamma_{\beta0}$ are used. First, the control variables $\beta$, $\gamma$ are set to 1 and all the counter variables $\Gamma_1$ to $\Gamma_{\beta0}$ are initialized to 0 (steps 400 and 401). Then, when it is time to inspect, the electrocardiogram data for the period INT2 is incorporated, the time-series data $BT_{k\gamma}$ of the characteristic quantity is calculated, then a subset $CT_{k\gamma}$ is calculated from the time-series data $BT_{k\gamma}$ and an average $\mu_{k\gamma}$ is calculated (steps 402 to 406). The processing in these steps 402 to 406 is the same as the processing in steps 301 to 305 in FIG. 3, but since different data for the period INT2 at a different inspection time is treated every time the control variable $\gamma$ changes, the sets $BT_{k\gamma}$, $CT_{k\gamma}$ and average value $\mu_{k\gamma}$ differ from one value of control variable $\gamma$ to another and a subscript $\gamma$ is affixed to indicate it.

When the average value $\mu_{k\gamma}$ is calculated in step 406, an average value deviation $Z_{x\gamma} = |\mu_{k\gamma}-\mu|$ is calculated (step 407). Then, for a comparison of $Z_{k\gamma}$ with each of the diagnostic levels L1 to L$\beta$0 (product of the set diagnostic coefficient and standard deviation), the processes of setting the control variable $\beta1$ to 1 (step 408) and incrementing the counter variable $\Gamma_{\beta1}$ by +1 when the deviation $Z_{k\gamma}$ is greater than the diagnostic level L$\beta$1 are carried out for $\beta1$ = 1 to $\beta0$ (steps 409 to 412). When these processes are completed (when the comparison result in step 411 is Yes), it is checked whether the control variable $\gamma$ has exceeded the number r of the differential sets as the inspection targets (step 413). If the control variable $\gamma$ has not exceeded the number r, the control variable $\gamma$ is incremented by +1 (step 414), the process returns to step 402, and if the control variable $\gamma$ has exceeded the number r, a diagnosis is made according to the values of the respective counter variables $\Gamma_1$ to $\Gamma_{\beta0}$, and the result is sent to the display control section 6 (step 415). Then, the overall process is repeated until the control variable $\beta$ exceeds the parameter Q2 (steps 416 and 417). As a example of processing in the above-described step 415, when the two diagnostic levels L1 and L2 are set to 3$\sigma$, 5$\sigma$ respectively ($\beta0$=2), a signal indicating "Attention" is sent to the display control section 6 if the number of times the level becomes L1 or greater is 20 or more when inspections are carried out r (= 50) times, while a signal indicating "Danger" is sent to the display control section 6 if the number of times the level becomes L2 or greater is 10 or more.

By the way, as in the case of FIG. 2, if the parameter Q2 is set to 1 in the processing of the variation detection section 4 and the diagnosis section 5 shown in FIG. 3 and FIG. 4, only one-time test can be carried out, and it is also possible to control so that iterations of inspection should be stopped or continued by deciding whether there is a stop instruction or not without using the control variable $\beta$. Furthermore, if one data acquisition period INT2 is, for example, 10 minutes in the processing in either FIG. 3 or FIG. 4, 700 characteristic quantities are obtained when the heart rate is 70/min. In the case, when an inspection is started at intervals of $\Delta T$ = 1 minute, for example, the target data acquisition period INT2 has an overlap as shown in FIG. 5. In such a case, the sum of elements on the overlapped section of adjacent two periods is available to calculate average values in step 305 or step 406 for the two corresponding different sets commonly, therefore when the overlapped section is large, use of the common sum can drastically improve the efficiency of the average value calculation processing.

Then, the processing of the display control section 6 will be explained using the flow chart in FIG. 6. When a diagnostic result is input through the processing shown in FIG. 3 or FIG. 4 (when the decision result in step 601 is Yes), the input result is displayed (step 602). Seceding processes after step 602 are carried out to make it possible to comprehend the condition of the patient self-explanatorily. In either case of FIG. 3 or FIG. 4, the difference set $CT_k$ is calculated at, for example, every 1-minute interval and an average value deviation $Z_k$ which is a difference between the average value of the elements of the set $CT_k$ and the average value of the ordinary distribution is calculated, and

therefore by incorporating these into a buffer in the display control section, displaying them along the time axis and displaying the electrocardiogram data itself as required, it is possible to observe not only the diagnostic result but also a time variation of the state of the patient. For this purpose, when no instruction to display the average value deviation is given from the operation apparatus 8 (when the decision result in step 603 is No), if the average value deviations are displayed on the display apparatus 7 so far, the displayed data are erased (step 604) and the process returns to step 601. But if there is an instruction to display the average value deviations, the average value deviations which are yet not displayed are added to the displayed data. Here, on the screen of the display apparatus 7, the average value deviations are shown on the vertical axis and a representative time indicating the period during which the average value deviation is calculated (e.g., inspection time in FIG. 3 and FIG. 4) are shown on the horizontal axis, and standard deviations of the ordinary distribution σ, or 2σ, ··· or the set decision levels, etc. on the vertical axis together. Since a new value of average value deviation is input every inspection cycle T2, for example, every one minute, if there is still an display area on the horizontal axis, the new value is added there and if there is no display area, the screen is scrolled so that the oldest data is removed and a new value is added. Furthermore, the display can be updated not only one data piece at a time but also a block of data pieces together. Such display of average value deviation allows the doctor to observe the condition of the patient in more detail.

Moreover, if any apparent abnormality is appreciated on the display data in step 605 by the doctor, and if the abnormal point on the screen is pointed by the mouse, it is interpreted as an instruction to display the electrocardiogram (step 606). When this instruction is given, the electrocardiogram data before and after the abnormal point are extracted from the recording apparatus 2 and displayed on the screen (step 607). This display time width may be preset in the control parameter setting section 10 or the display time width or time zone may be input when the screen is clicked. Furthermore, when an instruction for stopping the display of the electrocardiogram is given by clicking the operation apparatus 8 or a command box set on the screen (when the decision result in step 608 is Yes), the electrocardiogram which is being displayed is erased (step 609). This electrocardiogram display function allows the doctor to know the condition of the patient in further detail.

As explained above, the use of the diagnostic apparatus of the present invention shown in FIG. 1 should make it possible to monitor changes in characteristic quantities appearing in an electrocardiogram of the patient all the time, to detect abnormalities at an early stage automatically and to take necessary actions quickly to the changes in the condition of the disease. On the other hand, in the case of an analysis of the electrocardiogram, waveform analyses of an R wave, P wave, etc. cannot always be automatically performed with 100% accuracy and misjudges sometimes occur in individual waveform analyses though the frequency of misjudges is sufficiently small. However, since the apparatus of the present invention makes a diagnosis only by using statistical averaging about characteristic quantities, infrequent misjudges in waveform analyses will not affect the result, and the present invention makes it possible to make a diagnosis with much higher accuracy than the conventional arts which use short-time analyses. The same applies to general apparatuses other than apparatuses for electrocardiogram analyses.

Then, a more specific configuration of the apparatus in FIG. 1 will be explained. The apparatus in FIG. 1 is shown as an apparatus in which all components are put together as a single apparatus, but an electrocardiograph is used for measurement attached to the body of the patient all the time, while the display apparatus 7 and operation apparatus 8 need to be installed in places easily accessible to doctors, nurses or laboratory technicians, etc. Furthermore, since the ordinally distribution calculation section 3 need not always operate, installing the ordinary distribution calculation section 3 near each patient is not efficient, and the ordinary distribution calculation section 3 may be designed to be a program on a personal computer commonly available to a plurality of patients. The situation is similar for diagnoses of mechanical structures, etc.

FIG. 7 shows a system configuration example when these points are taken into consideration. Each measuring terminal of the system is provided with quantity of state measuring apparatuses 71a or 71b ···, communication interface 73a or 73b ··· and data collection/transfer apparatus 72a or 72b ···. The collection/transfer apparatus 72a or 72b collects measured values, applies processing such as noise elimination to these measured values if necessary, combine them into transmission data and send it to the communication interface 73a or 73b ···. The communication interface 73a or 73b send data to the center via a network 70. When a quantity of state measuring apparatus is fixed spatially, the quantity of state measuring apparatus and corresponding data collection/transfer apparatus may be connected via cables, but when a quantity of state measuring apparatus is always attached to patients as in the case of an electrocardiograph apparatus, they may be connected using a radio communication path. As the communication interfaces 73a, 73b, ···, interfaces connectable to the network 70 such as PHS terminals or modems for personal computers are used. In view of communication costs and the amount of data transferred, it is desirable to use stable and economical interfaces. As the data collection/transfer apparatuses 72a, 72b, ···, for example, personal computers can be used and when the quantity of state measuring apparatuses output analog measured values, it is also possible to design the data collection/transfer apparatuses in such a way as to digitize the analog values and incorporate the digital data. When the communication interfaces 73a, 73b, ··· and the network 70 have sufficient transmission capacities and at the same time communication fees depend on connect time, it is possible to combine collected data into files at certain

intervals and transfer the files in burst transfer mode and thereby reduce communication costs. As the network 70, a PHS channel network, public telephone network or the Internet can be used, or in the case of a system in a hospital, the LAN in the hospital may be used.

A recording apparatus 2, a ordinary distribution calculation section 3, a quantity of state detection section 4, a diagnosis section 5, a display control section 6, a display apparatus 7, an operation apparatus 8 and a control section 9 have functions similar to those in FIG. 1 and are installed in a center where doctors stay full time and connected to the network 70 via a communication interface 74. In this configuration, a control parameter setting section 10 performs settings such as timing for various measuring terminals. The ordinary distribution calculation section 3, the quantity of state detection section 4 and the diagnosis section 5, etc. need to be able to handle data from a plurality of measuring terminals. Each of these components can be constructed by a single unit if it allows time-sharing processing, but if time-sharing processing is not possible, each component may be constructed of a plurality of units and processing may be distributed. Such distributed processing can be controlled through usually used. Furthermore, the communication interface 74 needs to have a function of receiving all data from a plurality of measuring terminals. This function can be realized by providing a plurality of addresses corresponding to the network 70, or the function of sending a busy signal to let the communication partner wait, with providing an appropriate buffer. The configuration shown in FIG. 7 allows an accurate, early diagnosis of the condition of the patient staying at home who has a cardiac disease.

The configuration shown in FIG. 7 assumes that the digitalized quantity of state data itself is sent to the center via a network. This is preferable when it is desirable to observe the quantity of state data (hereinafter referred to as "electrocardiogram data") itself on the center side all the time. However, when full-time observation of the electrocardiogram data itself or its full-time application to other purposes is not performed on the center side, it is possible to prevent the electrocardiogram data from being sent all the time so as to alleviate the load of communications. FIG. 8 shows another configuration of the diagnostic apparatus of the present invention with these points taken into consideration. In this configuration, a measuring terminal 81 is provided with the functions of a quantity of state measuring apparatus 811, recording apparatus 812, ordinary distribution calculation section 813, state detection section 814 and diagnosis section 815. Other measuring terminals also have the same configuration.

With this configuration, the doctor, etc. uses an operation apparatus 801 of the center 80 to set control parameters of the measuring terminals 81, 82, ··· in a parameter setting section 808 of a control section 802. Then, through the control of the control section 802, the parameters of the measuring terminal 81, for example, are set in a control parameter setting section 818 of the control section 816 via a communication interface 803, network 83, communication interface 817. The same applied to the other measuring terminals. In each measuring terminal, ordinary distribution is calculated periodically, characteristic quantities are detected, and diagnosis according to these set parameters is made under the control of the control section 816, and the diagnostic result is sent to the center 80 via the network. Here the a diagnosis may be made every time one diagnostic data AT is processed as shown in FIG. 3. However, the diagnosis can also be made using a plurality of diagnostic data pieces as shown in FIG. 4. In the center 80, the data indicating the diagnostic result sent via the network is stored in a buffer 804 temporarily, read by a display control section 806 as appropriate and displayed on a display apparatus 807. The display apparatus in this case can be a display screen, a lamp which shoots errors or alarms or a speaker/buzzer, etc. which outputs an alarm sound. Anyway, it is desirable to display data in places where doctors and nurses stay full time. Furthermore, the diagnostic result data is saved in a recording apparatus 805 of the center 80 so that it may be traced later.

When a signal indicating abnormal sate, alarm, etc. is sent from some measuring terminal, or when the doctor, etc. considers it necessary, the doctor, etc. can input a command into the operation apparatus 801 so that the measuring terminal sends the electrocardiogram data itself or time-series data $\{Z_k\}$ of an average value deviation detected by the state detection section to the center. Now, assuming that this is a command for the measuring terminal 81, this command is sent through the network 83 to the control section 816 of the measuring terminal 81, then one or both of the electrocardiogram data from the recording apparatus 812 and time-series data $\{Z_k\}$ of an average value deviation from the state detection section 814 are extracted for a specified period under the control of the control section 816, sent through the communication interface 817, network 83, communication interface 803 and buffer 804 to the center 80, and stored into the recording apparatus 805. At this time, an average value $\mu$ and standard deviation $\sigma$ of the ordinary distribution calculated by the ordinary distribution calculation section 813 are also sent to the center 80 simultaneously and stored in the recording apparatus 805. Those data are further displayed on the display apparatus 807 under the control of the display control section 806. This display is a time-series display of the electrocardiogram data or average value deviation explained in steps 607 and 605 in FIG. 6. This allows the doctor, etc. to directly observe desired data and know a more detailed condition of the patient.

According to the above-described explanations in FIG. 8, the electrocardiogram data is sent to the center only when some abnormality occurs or when the doctor, etc. inputs a command, which allows the amount of information sent via a network to be reduced drastically. With this configuration, each measuring terminal calculates a ordinary distribution, detects the state or carries out diagnostic processing, and therefore there is no need to worry about the data processing capacity of the center even if the number of measuring terminals increases.

The present invention calculates a ordinary distribution indicating a variation in a quantity of state of an object, for example, a characteristic quantity of an electrocardiogram from data measured for many hours as a normal distribution, and compares, during a diagnosis, a difference between an average value of variations in an appropriate quantity of state, for example, a characteristic quantity of the electrocardiogram and the average value of the ordinary distribution with the standard deviation of the ordinary distribution automatically in a short time, and then makes a diagnosis, and can thereby obtain the following effects:

(1) The present invention can automatically monitor the quantity of state of an object all the time, automatically detect even any transitory change and take action in response to the change in an early stage. Especially, the present invention allows the doctor, etc. to comprehend changes appearing in the characteristic quantities of an electrocardiogram in an early stage, decide the necessity of giving the patient advice to prevent the advance of the disease, decide the content of the advice, decide the necessity for more detailed examinations, the necessity for cure or decide the content of the cure in an early stage.

(2) When noise is included when quantities of state of an object are acquired or when characteristic quantities cannot be detected correctly and an erroneous detection occurs, such noise and erroneous detection may affect diagnostic results. However, the present invention makes a diagnosis after carrying out statistical processing using data corresponding to more hours than the conventional arts, and can thereby make a consistently reliable, automatic diagnosis which is hardly affected by noise or erroneous detection.

(3) When a measuring location where quantities of state are collected is spatially distant from a center where the quantities of state are processed or results are used, the present invention adopts a configuration whereby the quantities of state collected on the measuring side or the diagnostic result obtained by processing the quantities of state are sent to the center through a network, and can thereby provide the effect of allowing the doctor, etc. who stays in a medical center or hospital to monitor all the time the condition of the heart of the patient having a cardiac disease who stays at home and comprehend changes in the condition of the patient at home in an early stage.

A diagnostic method for performing diagnosis according to the invention comprises the steps of incorporating electrocardiogram data measured by an electrocardiograph apparatus as for a given first period distribution calculation data, detecting characteristic quantities repeatedly appearing in the electrocardiogram data from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and calculating average value and standard deviation of said average value set by assuming that said average value set has normal distribution, and incorporating the electrocardiogram data measured by the electrocardiograph apparatus for a second period which is shorter than said first period as diagnostic data, detecting characteristic quantities repeatedly appearing in the electrocardiogram data from said diagnostic data to generate time-series data, generating a difference set whose elements consist of absolute values of differences between neighboring data pieces of said time-series data, and comparing the absolute value of the difference between the average value of the element of said difference set and the average value of said ordinary distribution with a diagnostic level obtained by multiplying a given diagnostic coefficient by said standard deviation.

Another diagnostic method for performing diagnosis according to the invention comprises the steps of incorporating electrocardiogram data measured by an electrocardiograph apparatus as for a given first period distribution calculation data, detecting characteristic quantities repeatedly appearing in the electrocardiogram data from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and calculating average value and standard deviation of said average value set by assuming that said average value set has normal distribution repeatedly setting a second period which is shorter than said first period with the passage of time, incorporating the electrocardiogram data measured by the electrocardiograph apparatus as diagnostic data for every second period, detecting characteristic quantities repeatedly appearing in the electrocardiogram data from each diagnostic data for to generate time-series data respectively, and generating difference sets each of which consists of absolute values of differences between neighboring data pieces of corresponding time-series data; and displaying an average value variation graphic that plots average values of the respective difference sets in association with times representing the second period corresponding to said difference sets together with a diagnostic level obtained by multiplying a given diagnostic coefficient by said standard deviation on a displaying means, and displaying, when an arbitrary time on this average value variation graphic is specified through an operating means, an electrocardiogram waveform showing a time variation of the electrocardiogram data in a time zone including said specified time on the displaying means so that said average value variation graphic or said electrocardiogram waveform is able to be observed for diagnosis.

Another diagnostic method for performing diagnosis according to the invention comprises the steps of incorporating electrocardiogram data measured by an electrocardiograph apparatus as for a given first period distribution calculation data, detecting characteristic quantities repeatedly appearing in the electrocardiogram data from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and calculating average value and standard deviation of said average value set by assuming that said average value set has normal distribution, repeatedly setting a second period which is shorter than said first period with the passage of time, incorporating the electrocardiogram data measured by the electrocardiograph apparatus as diagnostic data for every second period, detecting characteristic quantities repeatedly appearing in the electrocardiogram data from each diagnostic data to generate time-series data respectively, and generating difference sets each of which consists of absolute values of differences between neighboring data pieces of corresponding time-series data, and counting during a given third period the number of times this average value of each difference set exceeds a diagnostic level obtained by multiplying a given diagnostic coefficient by said standard deviation, and comparing said number of times with a given number of times for diagnosis.

In the above three diagnostic methods calculations of average value and standard deviation of said ordinary distribution from said distribution calculation data are repeated by periodically changing the period of incorporating said distribution calculation data and the average value and the standard deviation of said ordinary distribution are periodically updated.

Another diagnostic method for performing diagnosis of an object according to the invention comprises the steps of incorporating measured quantity of state of said object for a given first period as distribution calculation data, detecting characteristic quantities repeatedly appearing in said quantity of state from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and calculating average value and standard deviation of said average value set by assuming that said average value set has normal distribution; and incorporating measured quantity of state of said object for a second period which is shorter than said first period as diagnostic data, detecting characteristic quantities repeatedly appearing in said quantity of state from said diagnostic data to generate time-series data, generating a difference set whose elements consist of absolute values of differences between neighboring data pieces of said time-series data, and comparing the absolute value of the difference between the average value of the elements of said difference set and the average value of said ordinary distribution with a diagnostic level obtained by multiplying a given diagnostic coefficient by said standard deviation.

## Claims

**1.** A diagnostic apparatus comprising:

an electrocardiograph apparatus (1);
parameter setting means (10) for setting at least a first period, or a second period which is shorter than said first period, a positive integer parameter n, a positive integer parameter m and a diagnostic coefficient;
ordinary distribution calculating means (3) for incorporating electrocardiogram data measured by said electrocardiograph apparatus as distribution calculation data for said first period set in said parameter setting means, detecting characteristic quantities repeatedly appearing in the electrocardiogram from said distribution calculation data to generate time-series data, generating a difference set whose elements consist of absolute values of differences between neighboring data pieces of said time-series data, generating as many subsets of as parameter m set-in said parameter setting means each of which consists of n elements randomly collected from said difference set where n is said parameter set in said parameter setting means, calculating each average value of the elements of each subset to generate an average value set as a set having the ordinary distribution of said difference set, and calculating average value and standard deviation of said average value set by assuming that said average value set has normal distribution;
state detecting means (4) for incorporating the electrocardiogram data measured by said electrocardiograph

apparatus as diagnostic data for the second period set in said parameter setting means, detecting characteristic quantities repeatedly appearing in the electrocardiogram from said diagnostic data to generate time-series data, generating a difference set whose elements consist of absolute values of differences between neighboring data pieces of said time-series data, and calculating an average value of said difference set; and
diagnostic means (5) for performing diagnosis by comparing the absolute value of the difference between the average value calculated by said state detecting means and the average value of said ordinary distribution with a diagnostic level obtained by multiplying the diagnostic coefficient set in said parameter setting means by said standard deviation.

**2.** A diagnostic apparatus comprising:

an electrocardiograph apparatus (1);
parameter setting means (10) for setting at least a first period, or a second period which is shorter than said first period, a positive integer parameter n, a positive integer parameter m and a diagnostic coefficient;
ordinary distribution calculating means (3) for incorporating electrocardiogram data measured by said electrocardiograph apparatus as distribution calculation data for said first period set in said parameter setting means, detecting characteristic quantities repeatedly appearing in the electrocardiogram from said distribution calculation data to generate time-series data, generating a difference set whose elements consist of absolute values of differences between neighboring data pieces of said time-series data, generating as many subsets of as parameter m set in said parameter setting means each of which consists of n elements randomly collected from said difference set where n is said parameter set in said parameter setting means, calculating each average value of the elements of each subset to generate an average value set as a set having the ordinary distribution of said difference set, and calculating average value and standard deviation of said average value set by assuming that said average value set has normal distribution;
state detecting means (4) for repeatedly setting the second period set in said parameter setting means with the passage of time, incorporating the electrocardiogram data measured by said electrocardiograph apparatus as diagnostic data for every second period, detecting characteristic quantities repeatedly appearing in the electrocardiogram from each diagnostic data to generate time-series data respectively, generating difference sets each of which consists of absolute values of differences between neighboring data pieces of corresponding time-series data, and calculating an average value of each difference set; and
display controlling means (6) for displaying an average value variation graphic that plots average values of the respective difference sets calculated by said state detecting means in association with times representing said second period corresponding to said difference sets together with a diagnostic level obtained by multiplying a diagnostic coefficient set in said parameter setting means by said standard deviation on a displaying means, and displaying, when an arbitrary time on this average variation graphic is specified through an operating means, an electrocardiogram waveform indicating a time variation of the electrocardiogram data in a time zone including said specified time on the displaying means.

**3.** A diagnostic apparatus comprising:

an electrocardiograph apparatus (1);
parameter setting means (10) for setting at least a first period, or a second period which is shorter than said first period, third period, a positive integer parameter n, a positive integer parameter m, a diagnostic coefficient and the number of times for diagnosis;
ordinary distribution calculating means (3) for incorporating electrocardiogram data measured by said electrocardiograph apparatus as distribution calculation data for said first period set in said parameter setting means, detecting characteristic quantities repeatedly appearing in the electrocardiogram from said distribution calculation data to generate time-series data, generating a difference set whose elements consist of absolute values of differences between neighboring data pieces of said time-series data, generating as many subsets of as parameter m set in said parameter setting means each of which consists of n elements randomly collected from said difference set where n is said parameter set in said parameter setting means, calculating each average value of the elements of each subset to generate an average value set as a set having the ordinary distribution of said difference set, and calculating average value and standard deviation of said average value set by assuming that said average value set has normal distribution;
state detecting means (4) for repeatedly setting the second period set in said parameter setting means with the passage of time, incorporating the electrocardiogram data measured by said electrocardiograph apparatus as diagnostic data for every second period, detecting characteristic quantities repeatedly appearing in the electrocardiogram from each diagnostic data to generate time-series data respectively, generating difference

sets each of which consists of absolute values of differences between neighboring data pieces of corresponding time-series data, and calculating an average value of each difference set;
a counter for counting the number of times the average value of each difference set calculated by said state detecting means exceeds a diagnostic level obtained by multiplying a diagnostic coefficient set in said parameter setting means by said standard deviation during the third period set in said parameter setting means; and
diagnostic means (5) for performing diagnosis by comparing the count of said counter with the number of times for diagnosis set in said parameter setting means.

4. The diagnostic apparatus according to any one of claims 1 to 3, wherein said ordinary distribution calculating means is provided with functions of iterating calculations of the average value and standard deviation of said ordinary distribution by periodically changing the period of incorporating said distribution calculation data and periodically updating the average value and standard deviation of said ordinary distribution.

5. The diagnostic apparatus according to any one of claims 1 to 3, wherein said ordinary distribution calculating means and said state detecting means are constructed in such a way as to incorporate electrocardiogram data from said electrocardiograph apparatus via a network.

6. A diagnostic apparatus comprising a center apparatus (80) and-one or a plurality of measuring terminal apparatuses (81) connected to said center apparatus via a network (83), and each of said measuring terminal apparatuses comprising:

an electrocardiograph apparatus (811);
parameter setting means (818) for setting at least a first period or a second period which is shorter than said first period, a positive integer parameter n, a positive integer parameter m and a diagnostic coefficient;
ordinary distribution calculating means (813) for incorporating electrocardiogram data measured by said electrocardiograph apparatus as distribution calculation data for said first period set in said parameter setting means, detecting characteristic quantities repeatedly appearing in the electrocardiogram from said distribution calculation data to generate time-series data, generating a difference set whose elements consist of absolute values of differences between neighboring data pieces of said time-series data, generating as many subsets of as parameter m set in said parameter setting means each of which consists of n elements randomly collected from said difference set where n is said parameter set in said parameter setting means, calculating each average value of the elements of each subset to generate an average value set as a set having the ordinary distribution of said difference set, and calculating average value and standard deviation of said average value set by assuming that said average value set has normal distribution;
state detecting means (814) for repeatedly setting the second period set in said parameter setting means with the passage of time, incorporating the electrocardiogram data measured by said electrocardiograph apparatus as diagnostic data for every second period, detecting characteristic quantities repeatedly appearing in the electrocardiogram from each diagnostic data to generate time-series data respectively, generating difference sets each of which consists of absolute values of differences between neighboring data pieces of corresponding time-series data, and calculating an average value of each difference set; and
diagnostic means (815) for performing diagnosis by comparing the absolute value of the difference between the average value calculated by said state detecting means and the average value of said ordinary distribution with a diagnostic level obtained by multiplying the diagnostic coefficient set in said parameter setting means by said standard deviation;
a communication interface (817); and
controlling means (816) for controlling, during ordinary operation mode, so that the diagnostic result of said diagnostic means is sent to the center apparatus via said communication interface and network by operating said ordinary distribution calculating means, said state detecting means and said diagnostic means according to the parameters set in said control parameter setting means, and controlling, when a command is sent from the center apparatus, so that one or both of the electrocardiogram data from said electrocardiograph apparatus and the average value calculated by said state detecting means for every said second period are sent to the center apparatus via said communication interface and network according to the content of the command,

wherein said center apparatus comprising:

control parameter setting means (808) for setting control parameters used by each measuring terminal apparatus;
display controlling means (806) for controlling the displaying means;

a communication interface (803);
inputting means; and
controlling means (802) for sending parameters which are set in said control parameter setting means through said inputting means, to the corresponding measuring terminal apparatus via said communication interface and network so that said parameters are set in the control parameter setting means of said measuring terminal apparatus, and when a command is input from the inputting means to one of the measuring terminal apparatuses, sending said command to controlling means of said measuring terminal apparatus via said communication interface and network, controlling said display controlling means during ordinary operation mode so as to display the diagnostic result sent from each measuring terminal apparatus, and controlling said display controlling means, when one or both of said electrocardiogram data and time-series data of the average values are sent according to said command, so as to display the data.

**7.** A diagnostic program for allowing a computer to execute:

a first step of incorporating electrocardiogram data measured by an electrocardiograph apparatus for a given first period as distribution calculation data, detecting characteristic quantities repeatedly appearing in the electrocardiogram data from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and
calculating average value and standard deviation of said average value set by assuming that said average value set has normal distribution; and
a second step incorporating the electrocardiogram data measured by the electrocardiograph apparatus for a second period which is shorter than said first period of as diagnostic data, detecting characteristic quantities repeatedly appearing in the electrocardiogram data from said diagnostic data to generate time-series data, generating a difference set whose elements consist of absolute values of differences between neighboring data pieces of said time-series data, comparing the absolute value of the difference between the average value of said difference set and the average value of said ordinary distribution with a diagnostic level obtained by multiplying a given diagnostic coefficient by said standard deviation, and displaying the comparison result on a displaying means.

**8.** A diagnostic program for allowing a computer to execute:

a first step of incorporating electrocardiogram data measured by an electrocardiograph apparatus for a given first period as distribution calculation data, detecting characteristic quantities repeatedly appearing in the electrocardiogram data from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and
calculating average value and standard deviation of said average value set by assuming that said average value set has normal distribution;
a second step of repeatedly setting a second period which is shorter than said first period with the passage of time, incorporating the electrocardiogram data measured by the-electrocardiograph apparatus as diagnostic data for every second period, detecting characteristic quantities repeatedly appearing in the electrocardiogram data from each diagnostic data to generate time-series data respectively, and generating difference sets of which consists of absolute values of differences between neighboring data pieces of corresponding time-series data; and
a third step of displaying an average value variation graphic that plots average values of the respective difference sets in association with times representing the second period corresponding to said difference sets together with a diagnostic level obtained by multiplying a given diagnostic coefficient by said standard deviation on a displaying means, and displaying, when an arbitrary time on this average value variation graphic is specified through an operating means, an electrocardiogram waveform indicating a time variation of the electrocardiogram date in a time zone including said specified time on the displaying means.

**9.** A diagnostic program for allowing a computer to execute:

a first step of incorporating electrocardiogram data measured by an electrocardiograph apparatus for a given first period as distribution calculation data, detecting characteristic quantities repeatedly appearing in the electrocardiogram data from said distribution calculation data to generate time-series data, generating a difference set whose elements consist of absolute values of differences between neighboring data pieces of said time-series data, generating m subsets each of which consists of randomly collected n elements from said difference set (n, m: positive integers), calculating each average value of the elements of each subset to generate an average value set as a set having the ordinary distribution of said difference set, and
calculating average value and standard deviation of said average value set by assuming that said average value set has normal distribution;
a second step of repeatedly setting a second period which is shorter than said first period with the passage of time, incorporating the electrocardiogram data measured by the electrocardiograph apparatus as diagnostic data for every second period, detecting characteristic quantities repeatedly appearing in the electrocardiogram data from each diagnostic data to generate time-series data respectively, and generating difference sets each of which consists of absolute values of differences between neighboring data pieces of corresponding time-series data; and
a third step of counting during a given third period the number of times this average value of each difference set exceeds a diagnostic level obtained by multiplying a given diagnostic coefficient by said standard deviation, and displaying the result of comparing said number of times with a given number of times for diagnosis on a displaying means.

**10.** The diagnostic program according to any one of claims 7 to 9, wherein said first step includes the steps of repeating calculations of an average value and standard deviation of said ordinary distribution using said distribution calculation data by periodically changing the calculations of average value and standard deviation of said ordinary distribution from said distribution calculation data are repeated by periodically changing the period of incorporating said distribution calculation data and the average value and the standard deviation of said ordinary distribution are periodically updated.

FIG.1

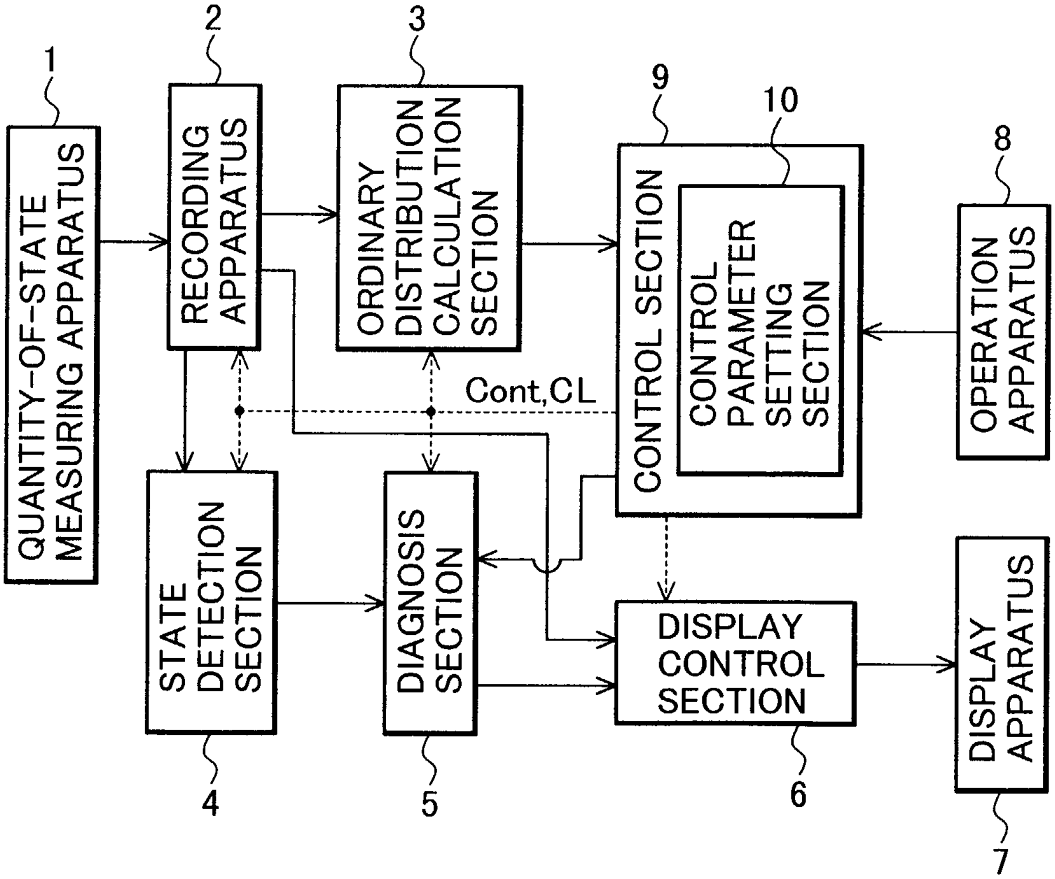
1
QUANTITY-OF-STATE MEASURING APPARATUS
2
RECORDING APPARATUS
3
ORDINARY DISTRIBUTION CALCULATION SECTION
9
CONTROL SECTION
10
CONTROL PARAMETER SETTING SECTION
8
OPERATION APPARATUS
Cont,CL
STATE DETECTION SECTION
4
DIAGNOSIS SECTION
5
DISPLAY CONTROL SECTION
6
DISPLAY APPARATUS
7

FIG.2

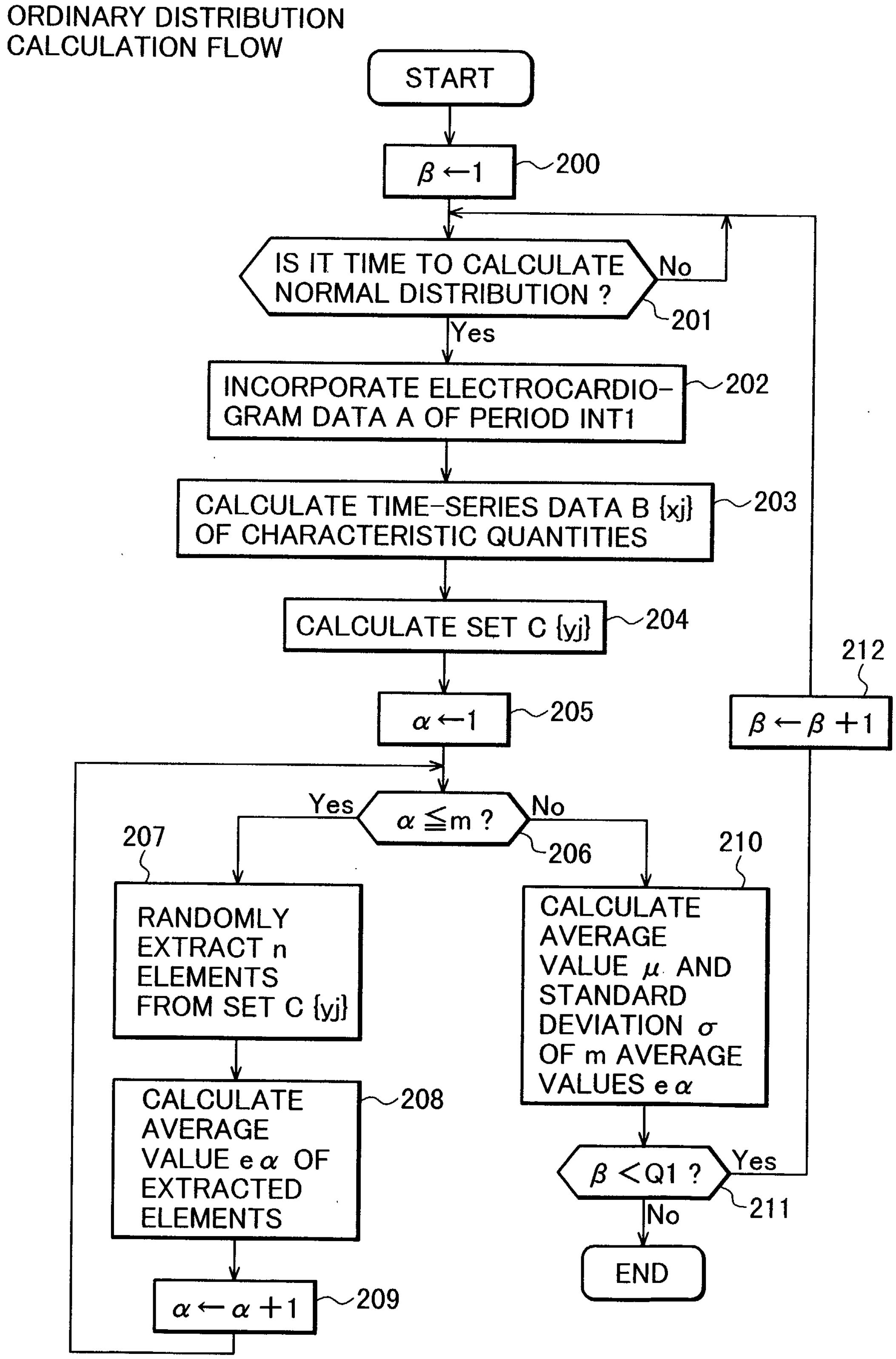
ORDINARY DISTRIBUTION CALCULATION FLOW
START
β←1
200
IS IT TIME TO CALCULATE NORMAL DISTRIBUTION ?
No
201
Yes
INCORPORATE ELECTROCARDIO-GRAM DATA A OF PERIOD INT1
202
CALCULATE TIME-SERIES DATA B {xj} OF CHARACTERISTIC QUANTITIES
203
CALCULATE SET C {yj}
204
212
β←β+1
α←1
205
Yes
α≦m ?
No
206
207
RANDOMLY EXTRACT n ELEMENTS FROM SET C {yj}
210
CALCULATE AVERAGE VALUE μ AND STANDARD DEVIATION σ OF m AVERAGE VALUES eα
CALCULATE AVERAGE VALUE eα OF EXTRACTED ELEMENTS
208
β<Q1 ?
Yes
211
No
END
α←α+1
209

FIG.3

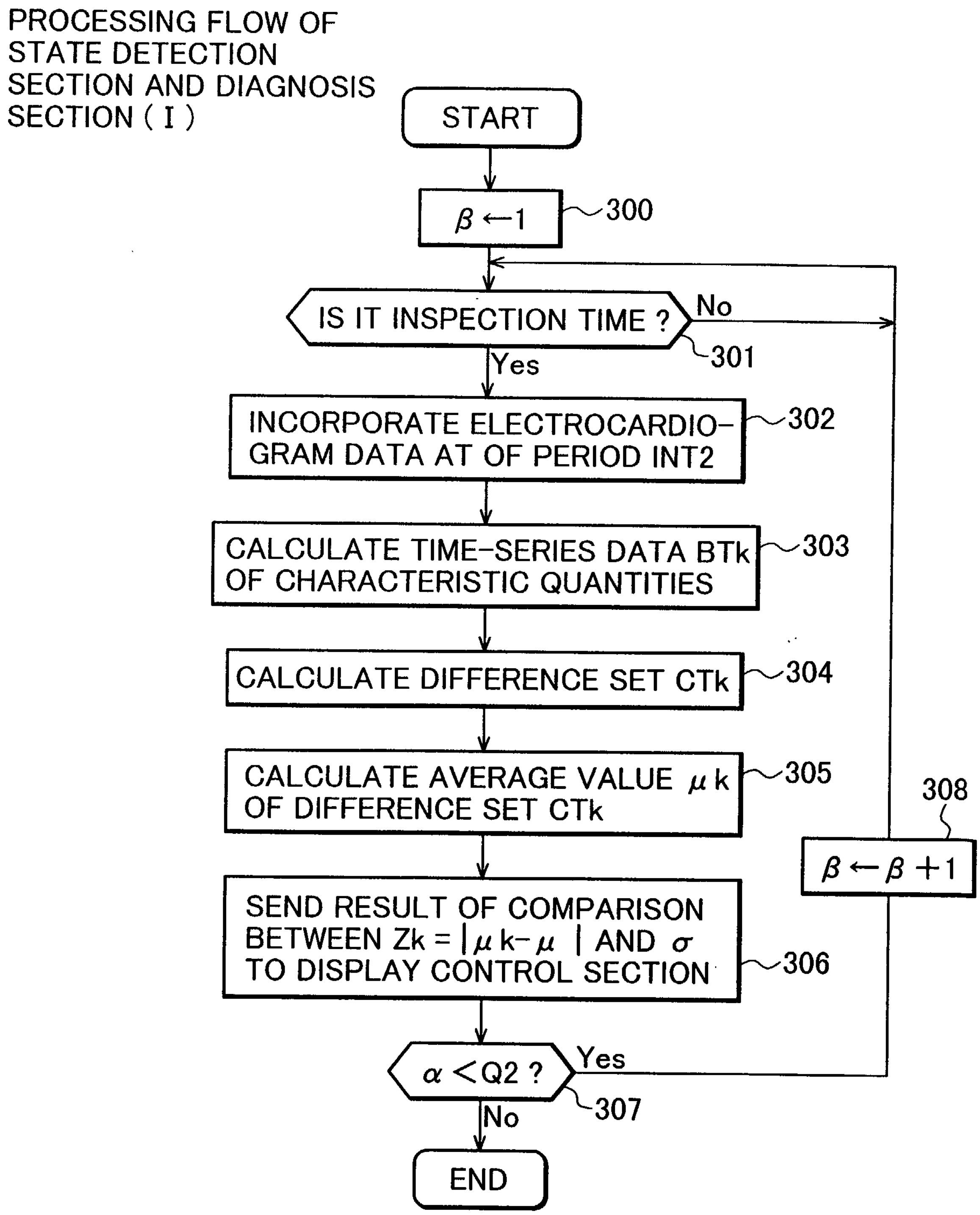
PROCESSING FLOW OF
STATE DETECTION
SECTION AND DIAGNOSIS
SECTION ( I )
START
β ←1
300
IS IT INSPECTION TIME ?
No
301
Yes
INCORPORATE ELECTROCARDIO-
GRAM DATA AT OF PERIOD INT2
302
CALCULATE TIME-SERIES DATA BTk
OF CHARACTERISTIC QUANTITIES
303
CALCULATE DIFFERENCE SET CTk
304
CALCULATE AVERAGE VALUE μ k
OF DIFFERENCE SET CTk
305
308
β ← β +1
SEND RESULT OF COMPARISON
BETWEEN Zk = | μ k− μ | AND σ
TO DISPLAY CONTROL SECTION
306
α < Q2 ?
Yes
307
No
END

FIG.4

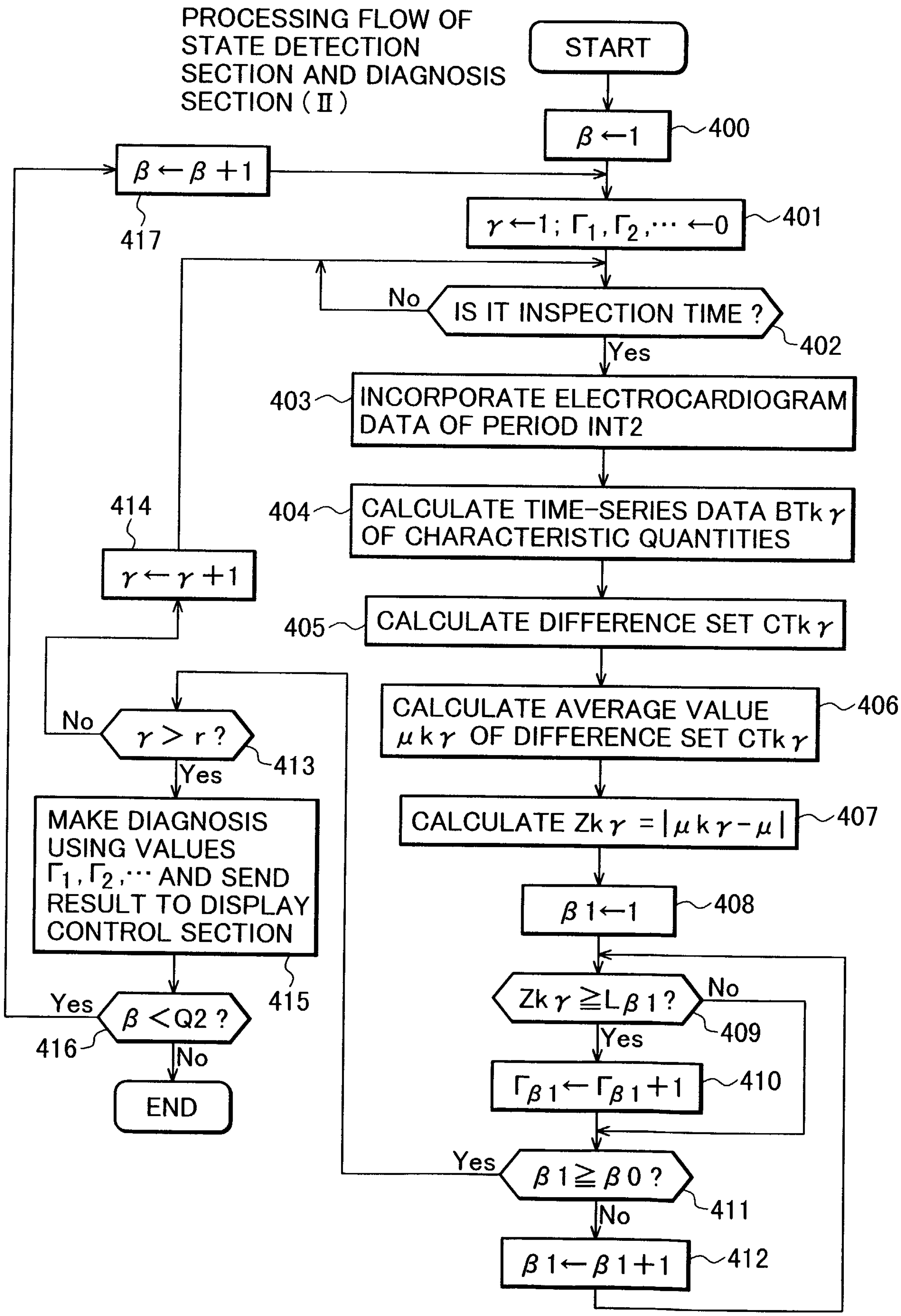
PROCESSING FLOW OF STATE DETECTION SECTION AND DIAGNOSIS SECTION (II)
START
β←1
400
β←β+1
417
γ←1; Γ1, Γ2, ··· ←0
401
No
IS IT INSPECTION TIME ?
402
Yes
403
INCORPORATE ELECTROCARDIOGRAM DATA OF PERIOD INT2
404
CALCULATE TIME-SERIES DATA BTkγ OF CHARACTERISTIC QUANTITIES
414
γ←γ+1
405
CALCULATE DIFFERENCE SET CTkγ
CALCULATE AVERAGE VALUE μkγ OF DIFFERENCE SET CTkγ
406
No
γ > r ?
413
Yes
MAKE DIAGNOSIS USING VALUES Γ1, Γ2, ··· AND SEND RESULT TO DISPLAY CONTROL SECTION
CALCULATE Zkγ = |μkγ−μ|
407
β1←1
408
415
Yes
β<Q2 ?
416
No
END
Zkγ ≧ Lβ1 ?
No
409
Yes
Γβ1←Γβ1+1
410
Yes
β1≧β0 ?
411
No
β1←β1+1
412

FIG.5

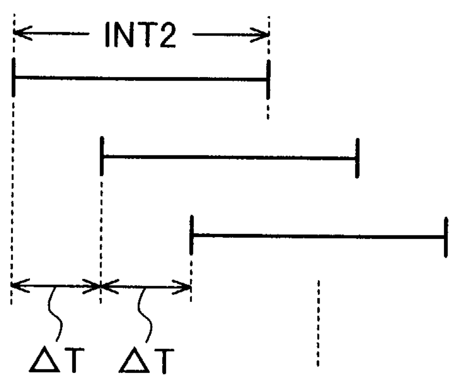

FIG.6

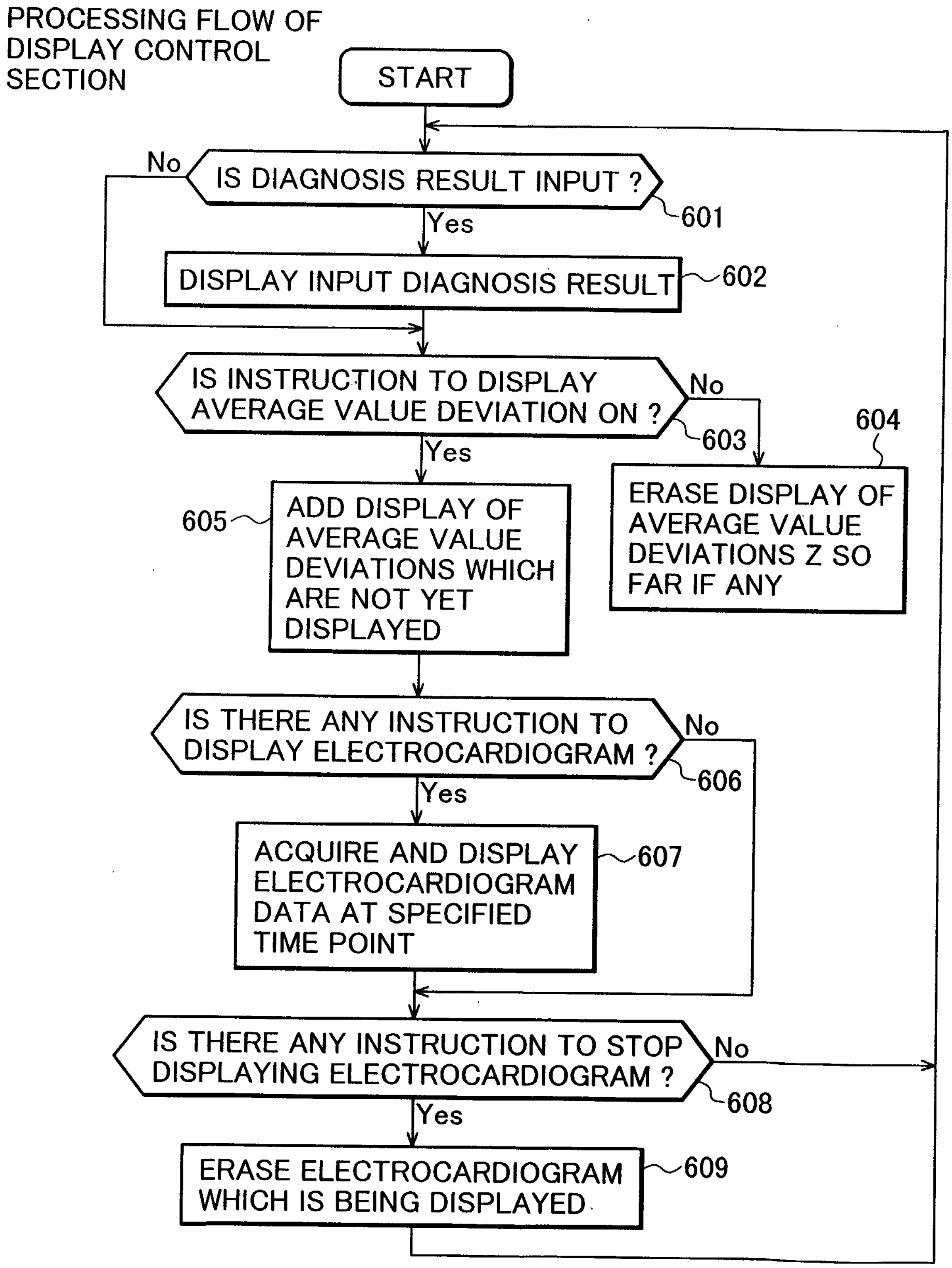
PROCESSING FLOW OF DISPLAY CONTROL SECTION
START
IS DIAGNOSIS RESULT INPUT ?
No
Yes
601
DISPLAY INPUT DIAGNOSIS RESULT
602
IS INSTRUCTION TO DISPLAY AVERAGE VALUE DEVIATION ON ?
No
Yes
603
604
ERASE DISPLAY OF AVERAGE VALUE DEVIATIONS Z SO FAR IF ANY
605
ADD DISPLAY OF AVERAGE VALUE DEVIATIONS WHICH ARE NOT YET DISPLAYED
IS THERE ANY INSTRUCTION TO DISPLAY ELECTROCARDIOGRAM ?
No
Yes
606
ACQUIRE AND DISPLAY ELECTROCARDIOGRAM DATA AT SPECIFIED TIME POINT
607
IS THERE ANY INSTRUCTION TO STOP DISPLAYING ELECTROCARDIOGRAM ?
No
Yes
608
ERASE ELECTROCARDIOGRAM WHICH IS BEING DISPLAYED
609

FIG.7

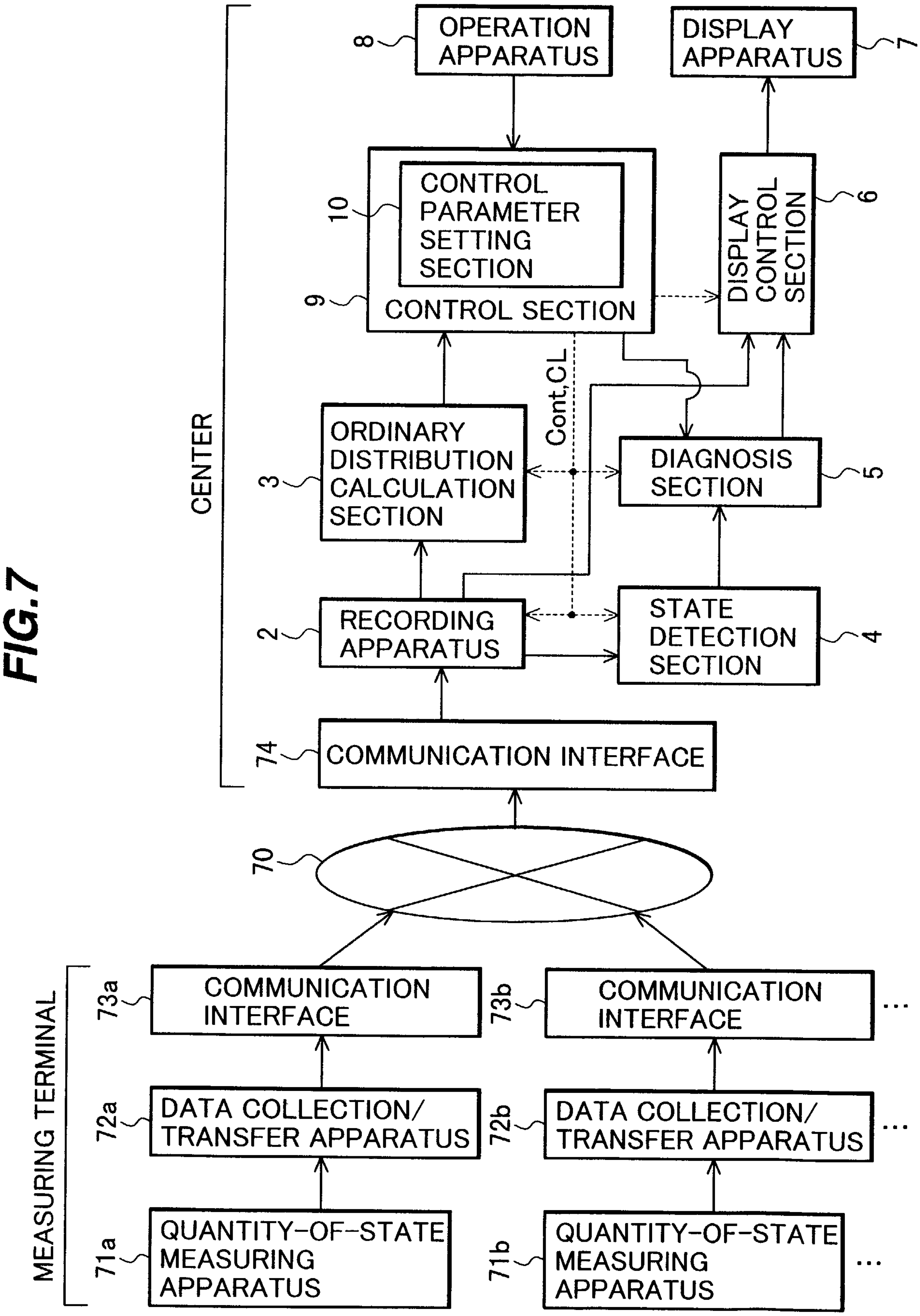
CENTER
8
OPERATION APPARATUS
DISPLAY APPARATUS
7
10
CONTROL PARAMETER SETTING SECTION
9
CONTROL SECTION
DISPLAY CONTROL SECTION
6
Cont,CL
3
ORDINARY DISTRIBUTION CALCULATION SECTION
DIAGNOSIS SECTION
5
2
RECORDING APPARATUS
STATE DETECTION SECTION
4
74
COMMUNICATION INTERFACE
70
MEASURING TERMINAL
73a
COMMUNICATION INTERFACE
73b
COMMUNICATION INTERFACE
72a
DATA COLLECTION/ TRANSFER APPARATUS
72b
DATA COLLECTION/ TRANSFER APPARATUS
71a
QUANTITY-OF-STATE MEASURING APPARATUS
71b
QUANTITY-OF-STATE MEASURING APPARATUS

FIG.8

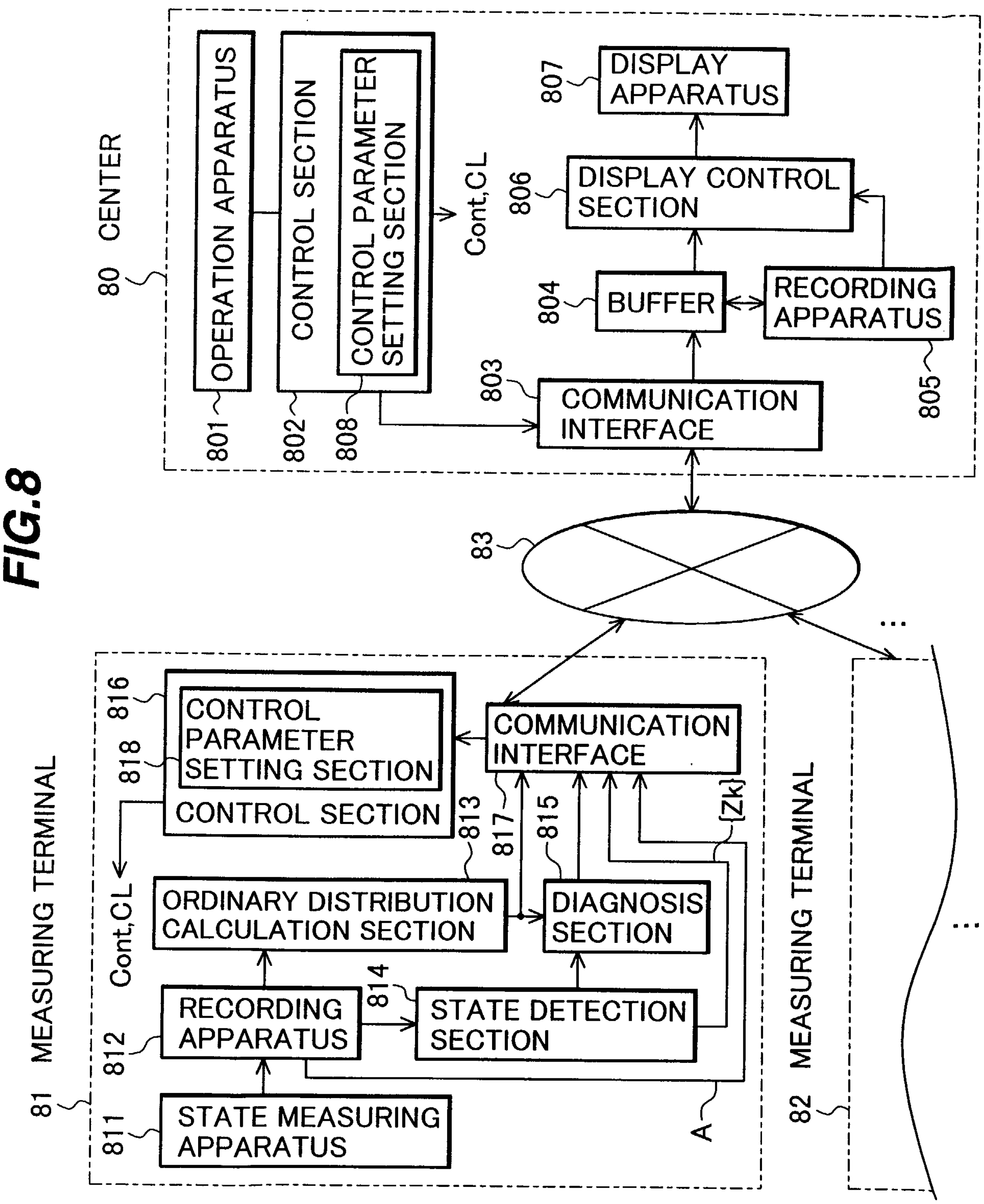
80 CENTER
OPERATION APPARATUS
801
CONTROL SECTION
802
CONTROL PARAMETER SETTING SECTION
808
Cont,CL
DISPLAY APPARATUS
807
DISPLAY CONTROL SECTION
806
BUFFER
804
RECORDING APPARATUS
805
COMMUNICATION INTERFACE
803
83
81 MEASURING TERMINAL
CONTROL PARAMETER SETTING SECTION
818
CONTROL SECTION
816
COMMUNICATION INTERFACE
Cont,CL
ORDINARY DISTRIBUTION CALCULATION SECTION
813
817
815
{Zk}
DIAGNOSIS SECTION
RECORDING APPARATUS
812
STATE DETECTION SECTION
814
STATE MEASURING APPARATUS
811
A
82 MEASURING TERMINAL

FIG.9

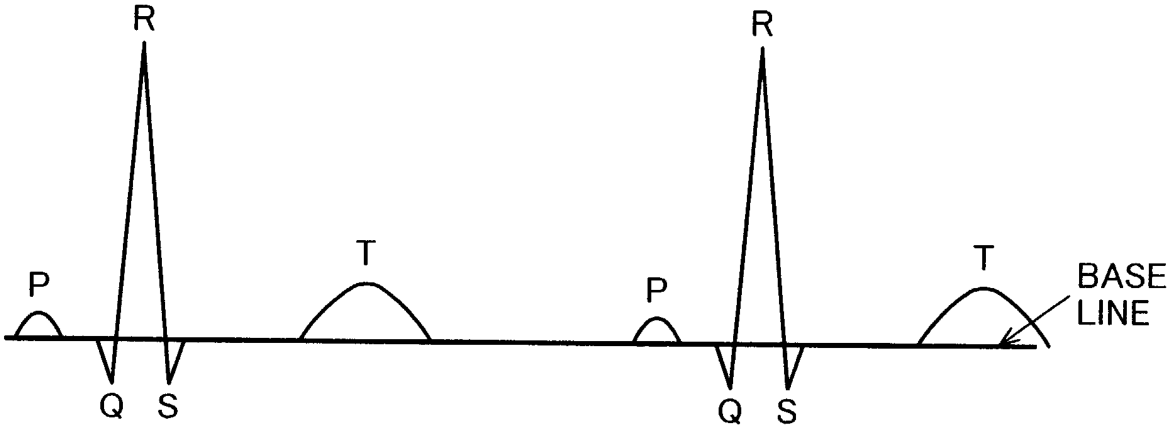


FIG.10A

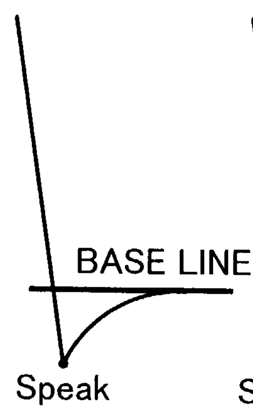


FIG.10B

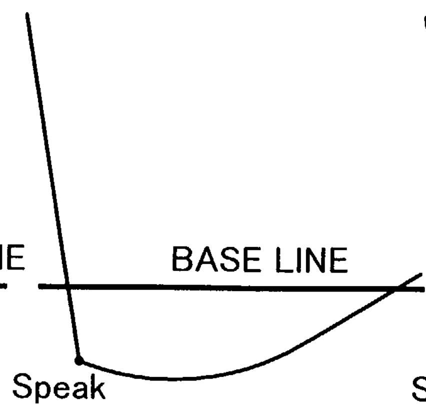


FIG.10C